(19) Europäisches Patentamt
European Patent Office
Office européen des brevets

(11) EP 2 439 197 A1

# (12) EUROPEAN PATENT APPLICATION

(43) Date of publication:
**11.04.2012 Bulletin 2012/15**

(21) Application number: **10013420.4**

(22) Date of filing: **07.10.2010**

(51) Int Cl.:
***C07D 235/16*** *(2006.01)* ***C07D 401/04*** *(2006.01)*
***C07D 401/12*** *(2006.01)* ***C07D 401/14*** *(2006.01)*
***C07D 403/04*** *(2006.01)* ***C07D 413/04*** *(2006.01)*
***A61K 31/4184*** *(2006.01)* ***A61P 1/08*** *(2006.01)*
***A61P 9/10*** *(2006.01)* ***A61P 25/24*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Santhera Pharmaceuticals (Schweiz) AG**
**4410 Liestal (CH)**

(72) Inventors:
- **Feurer, Achim**
  **79424 Auggen (DE)**
- **Soeberdt, Michael**
  **33605 Bielefeld (DE)**
- **Terinek, Miroslav**
  **4416 Bubendorf (CH)**
- **Nordhoff, Sonja**
  **52070 Aachen (DE)**
- **Siendt, Herve**
  **68220 Ranspach-le-Haut (FR)**
- **Weyermann, Philipp**
  **4450 Sissach (CH)**

(74) Representative: **Grünecker, Kinkeldey, Stockmair & Schwanhäusser Anwaltssozietät**
**Leopoldstrasse 4**
**80802 München (DE)**

## (54) Substituted benzimidazole derivatives as melanocortin 4 receptor antagonists

(57) The present invention relates to substituted benzimidazole derivatives as melanocortin-4 receptor (MC-4R) modulators.

EP 2 439 197 A1

## Description

### Field of the Invention

The present invention relates to substituted benzimidazole derivatives as melanocortin-4 receptor modulators. Depending on the structure and the stereochemistry, melanocortin-4 receptor modulators are either agonists or antagonists. The compounds of the invention are selective antagonists of the human melanocortin-4 receptor (MC-4R). The antagonists are useful for the treatment of disorders and diseases such as cachexia - induced by e.g. cancer, chronic kidney disease (CKD), chronic heart failure (CHF), or AIDS -, muscle wasting, anorexia - induced by e.g. chemotherapy or radiotherapy -, anorexia nervosa, amyotrophic lateral sclerosis (ALS), pain, neuropathic pain, anxiety and depression, nausea and emesis.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1R to MC-5R) have been identified and these are expressed in different tissues.

MC-1R was first found in melanocytes. Naturally occurring inactive variants of MC-1R in animals were shown to lead to alterations in pigmentation and a subsequent lighter coat color by controlling the conversion of phaeomelanin to eumelanin through the control of tyrosinase. From these and other studies, it is evident that MC-1R is an important regulator of melanin production and coat color in animals and skin color in humans.

The MC-2R is expressed in the adrenal gland representing the ACTH receptor. The MC-2R is not a receptor for α-MSH but is the receptor for the adrenocorticotropic hormone I (ACTHI).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask, et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators to near baseline levels. Recent studies have demonstrated that the anti-inflammatory actions of α-MSH are mediated by MC-1R. The mechanism by which agonism of MC-1R results in an anti-inflammatory response is likely through inhibition of the pro-inflammatory transcription activator, NF-κB. NF-κB is a pivotal component of the pro-inflammatory cascade, and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)).

Further evidence for the involvement of MC-Rs in obesity includes: 1) the agouti ($A^{vy}$) mouse which ectopically expresses an antagonist of the MC-1R, MC-3R and MC-4R is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R, and -5R agonist) injected intracerebroventricularly (ICV) in rodents, reduces food intake in several animal feeding models (NPY, ob/ob, agouti, fasted) while ICV injected SHU-9119 (MC-3R and 4R antagonist; MC-1R and -5R agonist) reverses this effect and can induce hyperphagia; 4) chronic intraperitoneal treatment of Zucker fatty rats with an α-NDP-MSH derivative (HP-228) has been reported to activate MC-1R, -3R, -4R, and -5R and to attenuate food intake and body weight gain over a 12 week period (I. Corcos et al., "HP-228 is a potent agonist of melanocortin receptor-4 and significantly attenuates obesity and diabetes in Zucker fatty rats," Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study," J. Urol., 160: 389-393, 1998). Activation of melanocortin receptors of the brain appears to cause normal stimulation of sexual arousal. Evidence for the involvement of MC-R in male and/or female sexual dysfunction is detailed in WO 00/74679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF) (Owen MJ and Nemeroff CB (1991) Physiology and pharmacology of corticotrophin releasing factor. Pharmacol Rev 43: 425-473). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Anxiolytic-Like and Antidepressant-Like Activities of MCL0129 (1-[(*S*)-2-(4-Fluorophenyl)-2-(4-isopropylpiperadin-1-yl) ethyl]-4- [4-(2-methoxynaphthalen-1-yl)butyl]piperazine), a Novel and Potent Nonpeptide Antagonist of the Melanocortin-4 Receptor; Shigeyuki Chaki et al, J. Pharm. Exp. Ther. (2003) 304(2), 818-26).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure.

Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (Marks D.L. et al. Role of the central melanocortin system in cachexia. Cancer Res. (2001) 61: 1432-1438).

Elevated levels of cytokines (e.g. leptin) are likely to be the cause of uremia-asscociated cachexia in patients with chronic kidney disease (CKD). It was shown that administration of agouti-related peptide (AgRP), an endogenous melanocortin-4 receptor inverse agonist, ameliorated uremic cachexia in mice with CKD. Gains in total body weight and lean body mass were observed along with increased food intake and lower basal metabolic rate. Furthermore, uremic cachexia in mice having a genetic MC4-R knockout was attenuated (Cheung W. et al. Role of leptin and melanocortin signaling in uremia associated cachexia. J. Clin. Invest. (2005) 115: 1659-1665). Intraperitoneal administration of small

molecule MC4-R antagonist NBI-12i to uremic mice resulted in similar findings (Cheung W. et al. Peripheral administration of the melanocortin-4 receptor antagonist NBI-12i ameliorates uremia-associated cachexia in mice. J. Am. Soc. Nephrol. (2007) 18: 2517-2524).

Rats with chronic heart failure (CHF) show an impaired ability to accumulate and maintain fat mass and lean body mass. Treatment of aortic banding induced CHF in rats with AgRP resulted in significantly increases in weight gain, lean body mass, fat accumulation, kidney weights and liver weights. (Batra A.K. et al. Central melanocortin blockage attenuates cardiac cachexia in a rat model of chronic heart failure. American Federation for Medical Research, 2008 Western Regional Meeting, abstract 379).

Radiation therapy in cancer patients has been associated with anorexia and nausea (Van Cutsem E., Arends J. The causes and consequences of cancer-associated malnutrition. Eur. J. Oncol. Nurs. (2005) 9 (Suppl 2): 51-63). In a model of radiation induced anorexia in mice, AgRP treated mice ate significantly more food than animals which underwent whole body radiation (RAD) and were vehicle treated. They showed a significantly reduced loss in weight compared to RAD mice treated with vehicle (Joppa M.A. et al. Central infusion of the melanocortin receptor antagonist agouti-related peptide (AgRP(83-132)) prevents cachexia-related symptoms induced by radiation and colon-26 tumors in mice. Peptides (2007) 28: 636-642)

Internal studies in ferrets have shown that a pharmacological inhibition of melanocortin-4 receptors strongly inhibits emesis. Accordingly, MC-4R inhibitors could be used to treat emesis, especially in patients undergoing chemotherapy.

Clinical observations indicate, that progression of Amytrophic Lateral Sclerosis (ALS) might be inversely correlated with body weight (e.g. Ludolph AC, Neuromuscul. Disord. (2006) 16 (8):530-8). Accordingly, MC-4R inhibitors could be used to treat ALS patients.

Experimental evidence in rats suggests the involvement of central MC4-R in the mechanism of development of tolerance and dependence following chronic morphine administration. Coadministration of the melanocortin-4 receptor antagonist HS014 during chronic morphine treatment delayed the developement of tolerance and prevented withdrawal hyperalgesia (Annasaheb S.K. et al. Central administration of selective melanocortin 4 receptor antagonist HS014 prevents morphine tolerance and withdrawal hyperalgesia. Brain Research (2007) 1181: 10-20).

Melanocortin-4-receptor modulators have been previously described in the literature. For example, substituted phenylpiperidine derivatives have been synthesized and explored as MC-4R agonists as well as antagonists.

WO 2005/056533 postulates that the benzimidazole and imidazopyridine derivatives described in the application are suitable for the treatment of pathological states and diseases involving one or more melanocortin receptors. Said application, however, does not provide any pharmacological data which would allow a person skilled in the art to evaluate the efficacy of the described compounds as melanocortin 4 receptor modulators.

In view of the unresolved deficiencies in treatment of various diseases and disorders as discussed above, it is an object of the present invention to provide novel compounds with improved ability to cross the blood brain barrier, which are useful as melanocortin-4 receptor antagonists to treat cachexia - induced by e.g. cancer, chronic kidney disease (CKD), chronic heart failure (CHF) or AIDS -, muscle wasting, anorexia - induced by e.g. chemotherapy or radiotherapy -, anorexia nervosa, amyotrophic lateral sclerosis (ALS), pain, neuropathic pain, anxiety and depression, nausea and emesis and other diseases with MC-4R involvement..

Surprisingly, it has been found that novel benzimidazoles according to formula (I) shown below solve the object of the present invention.

## Summary of the Invention

The present invention relates to substituted benzimidazole derivatives, of structural formula (I)

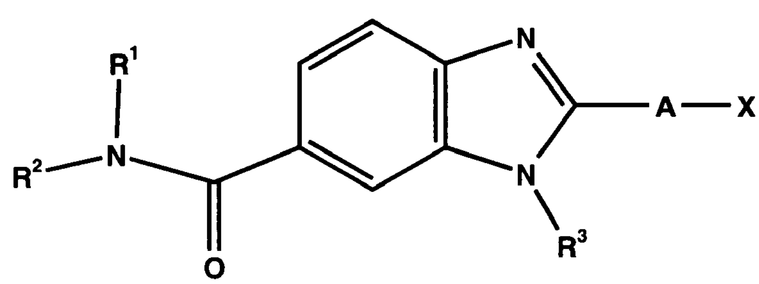

(I)

wherein $R^1$, $R^2$, $R^3$, A and X are defined as described below.

The benzimidazole derivatives of structural formula (I) are effective as melanocortin receptor modulators and are particularly effective as selective melanocortin-4 receptor (MC-4R) antagonists. They are therefore useful for the prophylaxis and treatment of disorders where the inactivation of the MC-4R is involved. The antagonists are useful for the prophylaxis and treatment of disorders and diseases such as cachexia - induced by e.g. cancer, chronic kidney disease (CKD), chronic heart failure (CHF) or AIDS -, muscle wasting, anorexia - induced by e.g. chemotherapy or radiotherapy -, anorexia nervosa, amyotrophic lateral sclerosis (ALS), pain, neuropathic pain, anxiety and depression, nausea and emesis. Thus, the present invention relates to compounds of formula (I) for use in the prophylaxis or treatment of a disorder, disease or condition responsive to the inactivation of the melanocortin-4 receptor in a mammal. More specific, the present invention relates to the prophylaxis or treatment of a disorder, disease of condition selected from cachexia, muscle wasting, anorexia, anxiety, depression, amyotrophic lateral sclerosis (ALS), pain, nausea and emesis.

Moreover, the present invention relates to a method for treating or preventing a disorder, disease or condition responsive to the inactivation of the melanocortin-4 receptor in a mammal comprising administering an effective dose of a compound according to formula (I) to a subject in need thereof. Preferably, the subject is a human.

The present invention also relates to pharmaceutical compositions comprising the compounds of the present invention and a pharmaceutically acceptable carrier.

**Detailed Description of the Invention**

The present invention relates to substituted benzimidazole derivatives useful as melanocortin receptor modulators, in particular, as selective MC-4R antagonists.

The compounds of the present invention are represented by structural formula (I)

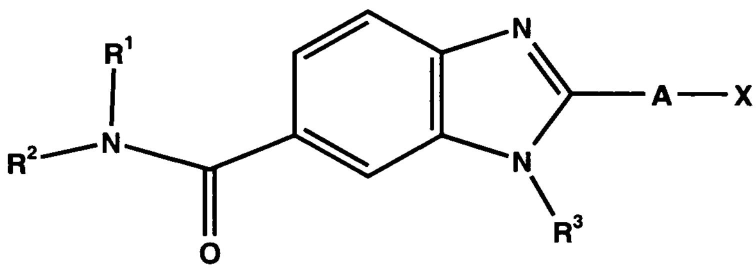

(I)

and enantiomers, diastereomers, tautomers, solvates and pharmaceutically acceptable salts thereof,
wherein
$R^1$ and $R^2$ are independently from each other selected from

H,
$C_{1-6}$alkyl,
$C_{1-6}$ alkylene-O-$C_{1-6}$alkyl,
$C_{1-3}$alkylene-heterocyclyl, and
$C_{1-6}$alkylene-$C_{3-7}$cycloalkyl, or

$R^1$ and $R^2$, together with the nitrogen atom to which they are attached to, form a 5 to 6- membered ring which may additionally contain one oxygen atom in the ring and which is unsubstituted or substituted by one or more substituents selected from OH, $C_{1-6}$alkyl, O-$C_{1-6}$alkyl, $C_{0-3}$alkylene-$C_{3-5}$cycloalkyl, $C_{1-6}$alkylene-O-$C_{1-6}$alkyl and $(CH_2)_{0-3}$-phenyl;
A is

-$C_{1-6}$alkylene,
-$C_{2-6}$alkenylene,
-$C_{2-6}$alkinylene or
a bond
wherein alkylene, alkenylene and alkinylene are unsubstituted or substituted with one ore more $R^4$, or two alkyl chains attached to the same carbon atom of the alkylene, alkenylene or alkinylene form a 3-6 membered ring;

$R^4$ is independently selected from

$C_{1-6}$alkyl,
O-$C_{1-6}$alkyl,
OH,
halogen, and
$C_{3-6}$ cycloalkyl;

X is 4 to 6-membered saturated heterocyclyl selected from the group consisting of $X^1$ to $X^7$

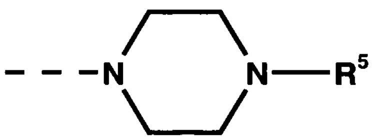

$X^1$,

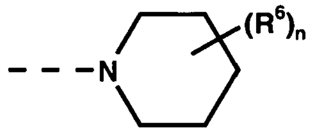

$X^2$,

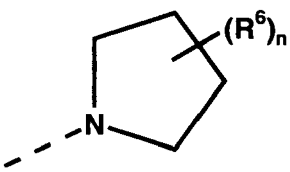

$X^3$,

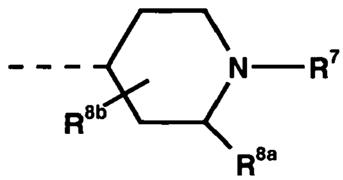

$X^4$,

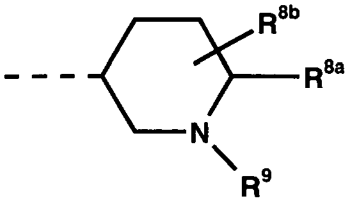

$X^5$,

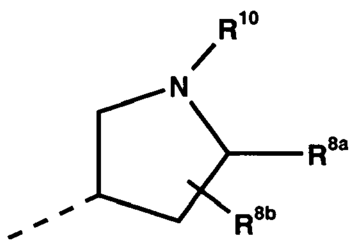

$X^6$

and

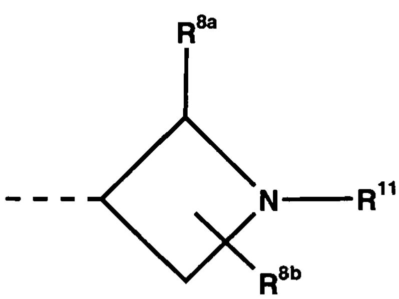

$X^7$,

5- to 6-membered heteroaryl selected from the group consisting of $X^8$ to $X^{13}$

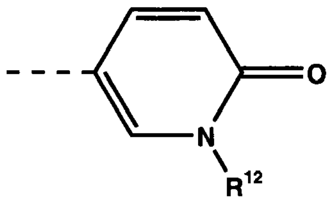 X8, 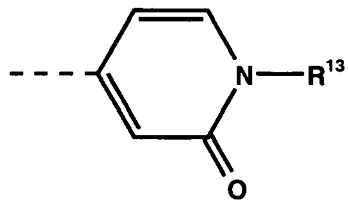 X9,

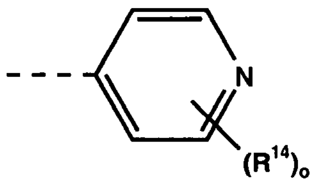 X10, 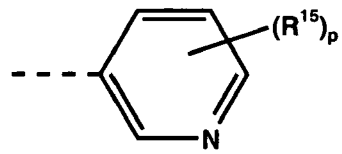 X11,

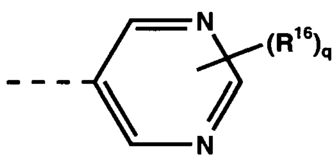 X12 and 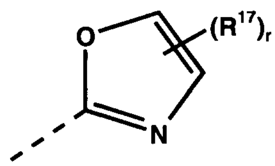 X13,

NH($C_{1-6}$alkyl),
N($C_{1-6}$alkyl)$_2$ or
$C_{3-8}$cycloalkyl, substituted with one or more $R^{18}$;

$R^5$ is H,

$C_{1-6}$ alkyl, unsubstituted or substituted with one or more substituents independently selected from OH, halogen and O-$C_{1-6}$alkyl,
C(O)-$C_{1-6}$alkyl,
COO$C_{1-6}$alkyl, or
4 to 6 membered saturated heterocycle having in the ring 1 or 2 heteroatoms independently selected from O, N and S;

$R^6$ is $C_{1-6}$ alkyl, unsubstituted or substituted with one or more substituents independently selected from OH, halogen, O-$C_{1-6}$alkyl, and C(O)-$C_{1-6}$alkyl,

or
4 to 6 membered saturated heterocycle containing in the ring 1 or 2 heteroatoms independently selected from O, N and S;

$R^7$ is H,

$C_{1-6}$ alkyl, unsubstituted or substituted with one or more substituents independently selected from OH, halogen and O-$C_{1-6}$alkyl,
C(O)-$C_{1-6}$alkyl,
COO$C_{1-6}$alkyl, or
4 to 6 membered saturated heterocycle containing in the ring 1 or 2 heteroatoms independently selected from O, N and S;

$R^{8a}$ and $R^{8b}$ are independently from each other

H,

$C_{1-6}$alkyl and
oxo;

$R^9$ is H,

$C_{1-6}$ alkyl, unsubstituted or substituted with one or more substituents independently selected from OH, halogen or
O-$C_{1-6}$alkyl,
C(O)-$C_{1-6}$alkyl,
COO$C_{1-6}$alkyl, or
4 to 6 membered saturated heterocycle containing in the ring 1 or 2 heteroatoms independently selected from O, N and S;

$R^{10}$ is H,

$C_{1-6}$alkyl, unsubstituted or substituted with one or more substituents independently selected from OH, halogen and
O-$C_{1-6}$alkyl,
C(O)-$C_{1-6}$alkyl,
COO$C_{1-6}$alkyl, or
4 to 6 membered saturated heterocycle containing in the ring 1 or 2 heteroatoms independently selected from O, N and S;

$R^{11}$ is H,

$C_{1-6}$alkyl, unsubstituted or substituted with one or more substituents independently selected from OH, halogen and
O-$C_{1-6}$alkyl,
C(O)-$C_{1-6}$alkyl,
COO$C_{1-6}$alkyl, or
4 to 6 membered saturated heterocycle containing in the ring 1 or 2 heteroatoms independently selected from O, N and S;

$R^{12}$ is H or

$C_{1-6}$alkyl, unsubstituted or substituted with one or more substituents independently selected from OH and F;

$R^{13}$ is H or

$C_{1-6}$alkyl, unsubstituted or substituted with one or more substituents independently selected from OH or F;

$R^{14}$ is O-$C_{1-6}$alkyl,

$C_{1-6}$alkyl or
$C_{3-6}$cycloalkyl;

$R^{15}$ is O-$C_{1-6}$alkyl,

$C_{1-6}$alkyl or
$C_{3-6}$cycloalkyl;

$R^{16}$ is OH,

O-$C_{1-6}$alkyl,
$C_{1-6}$alkyl or
$C_{3-6}$cycloalkyl;

$R^{17}$ is $C_{1-6}$alkyl or

$C_{3-6}$cycloalkyl;

$R^{18}$ is $NH_2$,

$NH(C_{1-6}alkyl)$, or
$N(C_{1-6}alkyl)_2$;

$R^3$ is $-CH_2CR^{19a}R1^{9b}CH_2$-T;
$R^{19a}$ and $R^{19b}$ are independently from each other selected from

H,
OH, and
halogen
or both together are =O;

T is

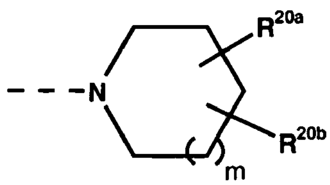
;

$R^{20a}$ and $R^{20b}$ are independently from each other selected from

H,
$C_{1-6}$alkyl and
$C_{3-6}$cycloalkyl;

m is 0 or 1;
n is 0, 1 or 2;
o is 1 or 2;
p is 1 or 2;
q is 0, 1 or 2 and
r is 0, 1 or 2.

In a preferred embodiment in combination with any of the embodiments above and below, the variants $R^1$ and $R^2$ independently from each other represent $C_{1-6}$alkyl or $C_{1-6}$alkylene-O-$C_{1-6}$ alkyl, wherein the alkyl chain is a straight chain or branched. More preferably, $R^1$ and $R^2$ represent a straight chain or branched $C_{1-6}$alkyl group and most preferably $R^1$ and $R^2$ represent a straight chain or branched $C_{3-6}$alkyl group.

In a preferred embodiment in combination with any of the embodiments above and below, the variant A represents a bond or methylene, wherein methylene is unsubstituted or substituted with one $R^4$ or wherein two hydrogen atoms of the methylene are replaced by a $-(CH_2)_s-$ bridge with s being an integer selected from 2, 3, 4 and 5.

$R^4$ is defined as above. In a preferred embodiment in combination with any of the embodiments above and below, $R^4$ represents $C_{1-6}$alkyl or halogen.

In a preferred embodiment in combination with any of the embodiments above and below, the variant X is a 4 to 6-membered saturated heterocyclyl selected from the group consisting of $X^1$, $X^2$ and $X^3$, a 5 to 6-membered heteroaryl selected from the group consisting of $X^8$ and $X^9$ or a $C_{3-8}$-cycloalkyl which is substituted with one or more $R^{18}$. In an equally preferred embodiment in combination with any of the embodiments above and below, the variant X is a 4 to 6-membered saturated heterocyclyl selected from the group consisting of $X^4$ and $X^7$.

In a preferred embodiment in combination with any of the embodiments above and below, the variant $R^5$ represents hydrogen or $C_{1-6}$alkyl which may be unsubstituted or substituted with one hydroxy group or alkoxy group.

In a further preferred embodiment in combination with any of the embodiments above and below, the variant $R^6$ represents $C_{1-6}$alkyl which may be unsubstituted or substituted with one hydroxy group or one C(O)-$C_{1-6}$alkyl.

In a preferred embodiment in combination with any of the embodiments above and below, the variant $R^7$ represents hydrogen, $C_{1-6}$alkyl which may be unsubstituted or substituted with one hydroxy group or one $COOC_{1-6}$alkyl, or a 4 to 6-membered saturated heterocycle containing in the ring 1 heteroatom selected from N and O. More preferably, $R^7$ represents hydrogen, $C_{1-3}$alkyl which may be unsubstituted or substituted with one hydroxy group, or $COOC_{1-4}$alkyl,

or a 4 to 5-membered saturated heterocycle containing in the ring 1 heteroatom selected from N and O.

In a preferred embodiment in combination with any of the embodiments above and below, the variants $R^{8a}$ and $R^{8b}$ independently from each other represent hydrogen or oxo.

In a preferred embodiment in combination with any of the embodiments above and below, the variant $R^9$ represents hydrogen or $C_{1-6}$alkyl which is unsubstituted.

In a further preferred embodiment in combination with any of the embodiments above and below, the variant $R^{10}$ represents hydrogen or $C_{1-6}$alkyl which is unsubstituted. More preferably, $R^{10}$ is hydrogen.

In a preferred embodiment in combination with any of the embodiments above and below, the variant $R^{11}$ represents hydrogen, $C_{1-6}$alkyl which is unsubstituted, or $COOC_{1-6}$alkyl.

In a preferred embodiment in combination with any of the embodiments above and below, the variants $R^{14}$ and $R^{15}$ independently from each other represent O-$C_{1-6}$alkyl, more preferably $OCH_3$.

In a preferred embodiment in combination with any of the embodiments above and below, the variant $R^{16}$ represents OH.

In a preferred embodiment in combination with any of the embodiments above and below, the variant $R^{17}$ represents $C_{1-6}$alkyl.

In a preferred embodiment in combination with any of the embodiments above and below, the variant $R^{18}$ represents $N(C_{1-6}alkyl)_2$.

In another preferred embodiment in combination with any of the above or below embodiments $R^3$ is $-CH_2CH_2CH_2-T$ or $-CH_2CHR^{19a}CH_2-T$ wherein $R^{19a}$ is OH or halogen. More preferably, $R^3$ represents $-CH_2CHR^{19a}CH_2-T$ wherein $R^{19a}$ is OH or halogen.

In a preferred embodiment in combination with any of the embodiments above and below, the variant T is defined as above wherein the index m is preferably 0.

The index n represents an integer selected from 0, 1 and 2. In a preferred embodiment in combination with any of the embodiments above and below, n represents the integers 0 and 1, more preferably, n is 0.

The index o represents an integer selected from 1 and 2. In a preferred embodiment in combination with any of the embodiments above and below, o is 1.

The index p represents an integer selected from 1 and 2. In a preferred embodiment in combination with any of the embodiments above and below, p is 1.

The index q represents an integer selected from 0, 1 and 2. In a preferred embodiment in combination with any of the embodiments above and below, q represents the integers 0 and 1, more preferably, q is 1.

The index r represents an integer selected from 0, 1 and 2. In a preferred embodiment in combination with any of the embodiments above and below, r represents the integers 0 and 1, more preferably, r is 0.

In a preferred embodiment in combination with any of the embodiments above and below, if $R^1$ and $R^2$ are independently from each otherH or $C_{1-6}$alkyl, $R^3$ is $-CH_2CH_2CH_2-T$, T is pyrrolidin or piperidin wherein T is unsubstituted or substituted with one $C_{1-6}$alkyl, A is a bond or $C_{1-6}$alkylene then X is not $X^4$, $X^5$, $X^6$, $X^7$, $X^{10}$, $X^{11}$, $X^{12}$ or $X^{13}$.

In the above and the following, the employed terms have the meaning as described below:

Alkyl is a straight chain or branched saturated hydrocarbon group. If the number of carbon atoms is not specified then alkyl is a straight chain or branched alkyl group having 1, 2, 3, 4, 5 or 6 carbon atoms. Examples of an alkyl group include methyl, ethyl, n-propyl, isopropyl, n-butyl, isobutyl, tert-butyl, n-pentyl, isopentyl, neopentyl, or n-hexyl.

Alkenyl is a straight chain or branched unsaturated hydrocarbon group having one to three double bonds, preferably one or two double bonds, most preferably one double bond. If the number of carbon atoms is not specified, then alkenyl is a straight chain or branched alkenyl group having 2, 3, 4, 5, or 6 carbon atoms. Preferred examples of a $C_{2-6}$alkenyl group are ethenyl, prop-1-enyl, prop-2-enyl, isoprop-1-enyl, n-but-1-enyl, n-but-2-enyl, n-but-3-enyl, isobut-1-enyl, isobut-2-enyl, n-pent-1-enyl, n-pent-2-enyl, n-pent-3-enyl, n-pent-4-enyl, n-pent-1,3-enyl, isopent-1-enyl, isopent-2-enyl, neopent-1-enyl, n-hex-1-enyl, n-hex-2-enyl, n-hex-3-enyl, n-hex-4-enyl, n-hex-5-enyl, n-hex-1,3-enyl, n-hex-2,4-enyl, n-hex-3,5-enyl, and n-hex-1,3,5-enyl. More preferred examples of a $C_{2-6}$alkenyl group are ethenyl and prop-1-enyl.

Alkinyl is a straight chain or branched unsaturated hydrocarbon group having one to three triple bonds, preferably one or two triple bonds, most preferably one triple bond. If the number of carbon atoms is not specified then alkinyl is a straight chain or branced alkinyl having 2, 3, 4, 5, or 6 carbon atoms. Preferred examples of a $C_{2-6}$alkinyl group are ethinyl, prop-1-inyl, prop-2-inyl, n-but-1-inyl, n-but-2-inyl, n-but-3-inyl, n-pent-1-inyl, n-pent-2-inyl, n-pent-3-inyl, n-pent-4-inyl, n-pent-1,3-inyl, isopent-1-inyl, neopent-1-inyl, n-hex-1-inyl, n-hex-2-inyl, n-hex-3-inyl, n-hex-4-inyl, n-hex-5-inyl, n-hex-1,3-inyl, n-hex-2,4-inyl, n-hex-3,5-inyl and n-hex-1,3,5-inyl. More preferred examples of a $C_{2-6}$alkinyl group are ethinyl and prop-1-inyl.

Cycloalkyl is a saturated hydrocarbon ring. If the number of carbon atoms is not specified then cycloalkyl has preferably 3, 4, 5, 6, 7 or 8 carbon atoms at the most. Examples of cycloalkyl are cyclopropyl, cyclobutyl, cyclopentyl, cyclohexyl, cycloheptyl or cyclooctyl. Preferred examples are cyclopropyl, cyclopentyl and cyclohexyl, most preferred is cyclopropyl.

Heteroaryl is an aromatic moiety having at least one heteroatom independently selected from O, N and/or S. If the number of ring atoms is not specified, then heteroaryl is a 5 or 6- membered monocyclic ring or 9 to 10-membered bicyclic ring systems. Examples of heteroaryl are thienyl, pyrrolyl, imidazolyl, pyrazolyl, pyridyl, pyrazinyl, pyrimidinyl, pyridazinyl, isothiazolyl, isoxazyl, furanyl, indazolyl, benzofuranyl, indolyl, quinolinyl, isoquinoninyl and purinyl. More preferred examples are thienyl, furanyl, imidazolyl, pyridyl, and pyrimidinyl.

Heterocyclyl is a saturated or unsaturated ring containing at least one heteroatom independently selected from O, N and/or S. If the number of ring atoms is not specified then heterocyclyl is 4 to 8-membered monocyclic ring or 9 to 10-membered bicyclic ring system. In a bicyclic ring system, one of the rings may be a saturated ring and the other an aromatic ring. Examples of heterocyclyl are tetrahydrofuranyl, azetidinyl, pyrrolidinyl, pyrrolinyl, piperidinyl, piperazinyl, pyranyl, morpholinyl, thiomorpholinyl, tetrahydropyranyl, 1,4-dioxanyl, azepanyl, oxepanyl, or chromanyl. Preferred examples are azetidinyl, piperazinyl, piperidinyl and pyrrolidinyl.

Halogen is a halogen atom selected from F, Cl, Br and I, preferably from F, Cl and Br.

The compounds of structural formula (I) are effective as melanocortin receptor modulators and are particularly effective as selective modulators of MC-4R. They are useful for the treatment and/or prevention of disorders responsive to the inactivation of MC-4R, such as cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis and other diseases with MC-4R involvement.

**Optical Isomers - Diastereomers - Geometric Isomers - Tautomers**

Compounds of structural formula (I) contain one or more asymmetric centers and can occur as racemates and racemic mixtures, single enantiomers, diastereomeric mixtures and individual diastereomers. The present invention is meant to comprehend all such isomeric forms of the compounds of structural formula (I).

Compounds of structural formula (I) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formula (I) may be obtained by stereospecific synthesis using optically pure starting materials or reagents of known absolute configuration.

**Salts**

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylamino-ethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, furnaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

## Utility

Compounds of formula (I) are melanocortin receptor antagonists and as such are useful in the treatment, control or prevention of diseases, disorders or conditions responsive to the inactivation of one or more of the melanocortin receptors including, but not limited to, MC-1 R, MC-2R, MC-3R, MC-4R or MC-5R. Such diseases, disorders or conditions include, but are not limited to, cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis.

The compounds of formula (I) can be further used in the treatment, control or prevention of hypertension, hyperlipidemia, osteoarthritis, cancer, gall bladder disease, sleep apnea, compulsion, neuroses, insomnia/sleep disorder, substance abuse, pain, fever, inflammation, immune-modulation, rheumatoid arthritis, skin tanning, acne and other skin disorders, neuroprotective and cognitive and memory enhancement including the treatment of Alzheimer's disease.

In another embodiment in combination with any of the above or below embodiments the compound of formula (I) can be used as a melanocortin-4 receptor antagonist.

In another embodiment in connection with any of the above or below embodiments the compound of formula (I) can be used for the prophylaxis or treatment of disorders, diseases or conditions responsive to the inactivation of the melanocortin-4 receptor in a mammal.

In another embodiment in connection with any of the above or below embodiments the compound of formula (I) can be used for the prophylaxis or treatment of a disorder, disease or condition selected from cachexia, muscle wasting, anorexia, anxiety, depression, amyotrophic lateral sclerosis (ALS), pain, nausea and emesis.

In another embodiment in connection with any of the above or below embodiments the compound of formula (I) can be used for the prophylaxis or treatment of cachexia selected from cancer cachexia, cachexia induced by chronic kidney disease (CKD), cachexia induced by chronic heart failure (CHF) or cachexia induced by AIDS.

In another embodiment in connection with any of the above or below embodiments the compound of formula (I) can be used for the prophylaxis or treatment of anorexia selected from anorexia nervosa or anorexia induced by radiotherapy or chemotherapy.

In another embodiment in connection with any of the above or below embodiments the compound of formula (I) can be used for the prophylaxis or treatment of pain, in particular neuropathic pain.

In another embodiment in connection with any of the above or below embodiments the compound of formula (I) can be used for the prophylaxis or treatment of cancer cachexia.

In another embodiment in connection with any of the above or below embodiments the compound of formula (I) can be used for the prophylaxis or treatment of muscle wasting.

In another embodiment in connection with any of the above or below embodiments the compound of formula (I) can be used for the prophylaxis or treatment of anorexia.

In another embodiment in connection with any of the above or below embodiments the compound of formula (I) can be used for the prophylaxis or treatment of anxiety and/or depression.

In another embodiment in connection with any of the above or below embodiments the compound of formula (I) can be used for the prophylaxis or treatment of emesis.

In another embodiment in connection with any of the above or below embodiments the compound of formula (I) can be used for the prophylaxis or treatment of amytrophic lateral sclerosis (ALS).

## Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably compounds of formula (I) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cachexia induced by e.g. cancer, chronic kidney disease (CKD) or chronic heart failure (CHF), muscle wasting, anorexia induced by e.g. chemotherapy or radiotherapy, anorexia nervosa, amyotrophic lateral sclerosis (ALS), pain, neuropathic pain, anxiety and depression, nausea and emesis generally satisfactory results are obtained when the compounds of the present invention are administered at a daily dosage of from about 0.001 milligram to about

100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

## Formulation

The compounds of formula (I) are preferably formulated into a dosage form prior to administration. Accordingly the present invention also includes a pharmaceutical composition comprising a compound of formula (I) and a suitable pharmaceutical carrier. The present pharmaceutical compositions are prepared by known procedures using well-known and readily available ingredients. In making the formulations of the present invention, the active ingredient (a compound of formula (I)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

## Preparation of Compounds of the Invention

The compounds of formula (I) when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formula (I) may be obtained by stereospecific synthesis using optically pure starting materials or reagents of known configuration.

The compounds of formula (I) of the present invention can be prepared according to the procedures of the following Schemes and Examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The Examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate, sodium hydroxide and potassium hydroxide, and extraction of the liberated amine free base into an organic solvent followed by evaporation. The amine free base isolated in this manner can be further converted into another pharmaceutically acceptable salt by dissolution in an organic solvent followed by addition of the appropriate acid and subsequent evaporation, precipitation or crystallization. All temperatures are degrees Celsius.

In the schemes, preparations and examples below, various reagent symbols and abbreviations have the following meanings

| | |
|---|---|
| DCM | dichloromethane |
| DIEA | ethyl-diisopropylamine |
| DMAP | 4-(Dimethylamino)pyridine |
| DMF | N,N-dimethylformamide |
| d | day |

| | |
|---|---|
| EDC | 1-(3-dimethylaminopropyl)-3-ethylcarbodiimide hydrochloride |
| $Et_2O$ | diethyl ether |
| EtOH | ethanol |
| EtOAc | ethylacetate |
| h | hour(s) |
| NMM | N-methylmorpholine |
| THF | tetrahydrofurane |
| $t_R$ (min) | HPLC retention time |

**Reaction Scheme 1:**

**Synthesis of N-substituted 3,4-diamino-benzoic acid amides**

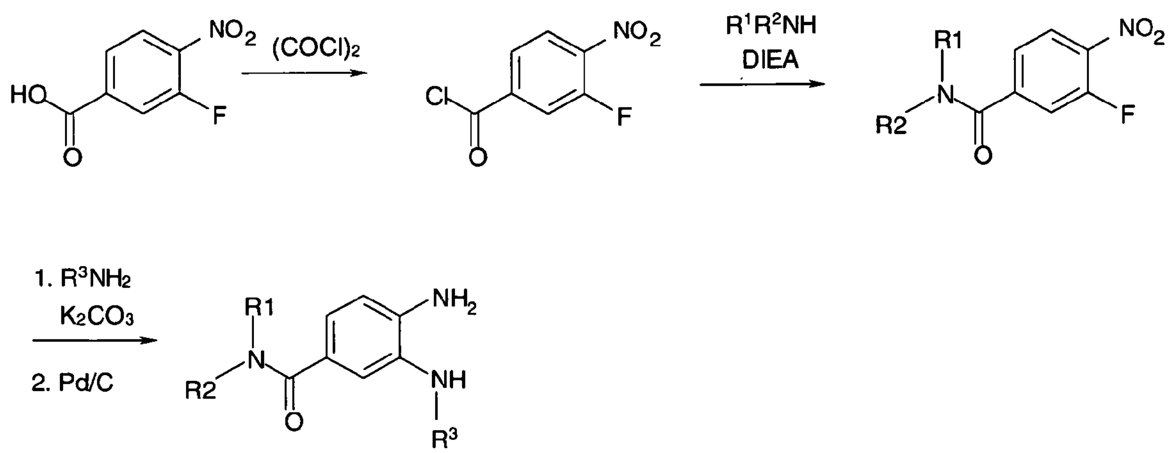


As shown in Reaction Scheme 1, secondary amines $R^1R^2NH$ are reacted in the presence of a suitable base, e.g. DIEA, with 3-fluoro-4-nitrobenzoylchloride - obtained from 3-fluoro-4-nitro benzoic acid via standard acid chloride formation with e.g. oxalylchloride - to obtain the corresponding disubstituted amides. Nucleophlic aromatic substitution of the fluoro group by a primary amine $R^3NH_2$ under basic conditions and subsequent reduction of the nitro group with e.g. palladium on charcoal in a suitable solvent or solvent mixture, e.g. ethanol and ethylacetate, delivers the N-substituted 3,4-diamino-benzoic acid amides.

**Reaction Scheme 2:**

**Benzimidazole synthesis**

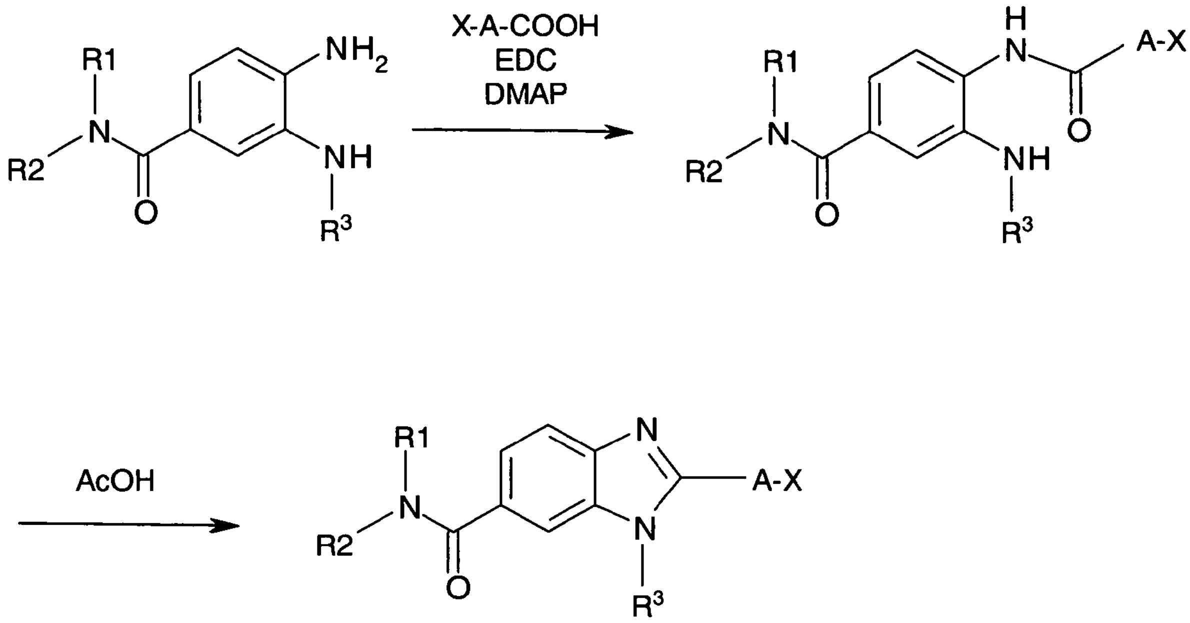


The reaction product from Reaction scheme 1 can be reacted with a carboxylic acid X-A-COOH under standard amide coupling conditions, e.g. EDC and DMAP, in a suitable solvent, e.g. dichloromethane, to deliver the corresponding 4-acylamino-3-(substituted amino)-benzoic acid amide. The acyl formation occurs selectively at the 4-position as this is activated (compared to the 3 position) due to the para-standing dialkylamino-acyl group. The reaction product can be heated in acetic acid to yield the benzimidazole derivatives according to reaction scheme 2.

**Reaction Scheme 3:**

**Benzimidazole synthesis**

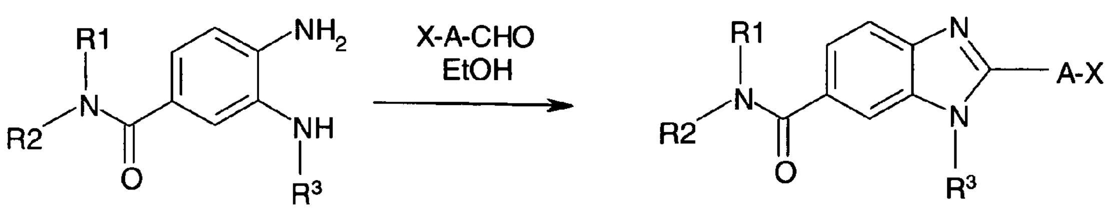


Alternatively, benzimidazoles can be obtained by reaction of the product of reaction scheme 1 with an aldehyde X-A-CHO in a suitable solvent, e.g. ethanol to yield benzimidaoles according to reaction scheme 3.

**Reaction Scheme 4:**

**Benzimidazole synthesis**

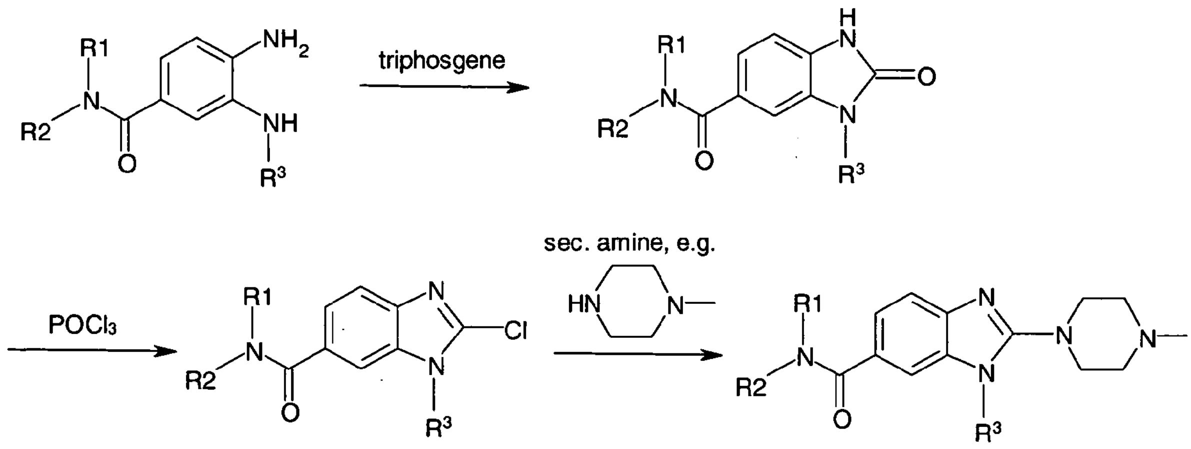


A route to 2-(substituted amino)-substituted benzimidazoles is shown in reaction scheme 4. The product of the sequence according to reaction scheme 1 is reacted with triphosgene to yield the 2-oxo-1,3-dihydrobenzimidazole derivative which can be transformed to the corresponding 2-chloro benzimidazole derivative by reaction with phosphorylchloride $POCl_3$. The chloro atom of this product can subsequently be replaced by an amine nitrogen, e.g. by reaction with a secondary cyclic amide like e.g. N-methylpiperazine.

**Analytical LC-MS**

The compounds of the present invention according to formula (I) were analyzed by analytical LC-MS. The conditions are summarized below.

<u>Analytical conditions summary:</u>

LC10Advp-Pump (Shimadzu) with SPD-M10Avp (Shimadzu) UV/Vis diode array detector and QP2010 MS-detector (Shimadzu) in ESI+ modus with UV-detection at 214, 254 and 275 nm,
Column: Waters XTerra MS C18, 3.5 μm, 2.1 * 100 mm,
linear gradient with acetonitrile in water (0.15% HCOOH)
Flow rate of 0,4 ml/min;

| | |
|---|---|
| Mobile Phase A: | water (0.15% HCOOH) |
| Mobile Phase B: | acetonitrile (0.15% HCOOH) |

Methods are:
**A:**

start concentration 10% acetonitrile

| | | |
|---|---|---|
| 10.00 | B.Conc | 60 |
| 11.00 | B.Curve | 2 |
| 12.00 | B.Conc | 99 |
| 15.00 | B.Conc | 99 |
| 15.20 | B.Conc | 10 |
| 18.00 | Pump STOP | |

**B:**

start concentration 15% acetonitrile

| | | |
|---|---|---|
| 12.00 | B.Conc | 99 |
| 15.00 | B.Conc | 99 |

(continued)

start concentration 15% acetonitrile

| | | |
|---|---|---|
| 15.20 | B.Conc | 15 |
| 18.00 | STOP 0 | |

**C:**

linear gradient from 5% to 95% acetonitrile in water (0.15% HCOOH)

| | |
|---|---|
| 0.00 min | 5% B |
| 5.00 min | 95% B |
| 5.10 min | 99% B |
| 6.40 min | 99% B |
| 6.50 min | 5% B |
| 8.00 min | Pump STOP |

The following tables describe the distinct examples of the invention which can be prepared via the reaction schemes 1 to 4.

**Table 1:**

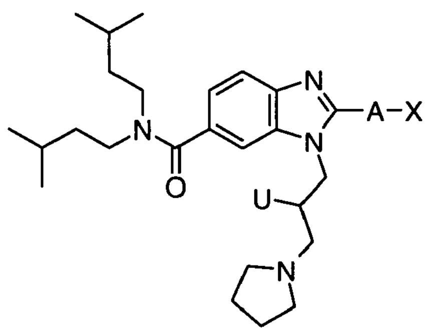

| | | | | HPLC | | MS | |
|---|---|---|---|---|---|---|---|
| No. | salt | A-X | U | $t_R$ (min) | method | MW (calc.) free base | $[M+H]^+$ (found) |
| 1 | citrate | 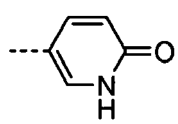 | -OH | 5.18 | A | 521.70 | 523 |
| 2 | HCOOH | 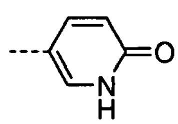 | -F | 5.98 | A | 523.69 | 524 |
| 3 | 2x HCOOH | 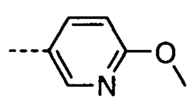 | -OH | 6.57 | A | 535.73 | 536 |
| 4 | citrate | 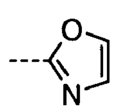 | -OH | 6.41 | A | 495.66 | 496 |

**Table 2:**

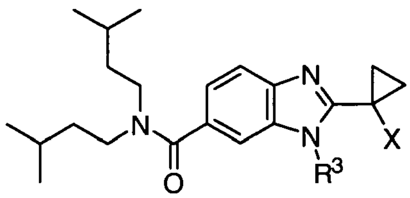

| | | | | HPLC | | MS | |
|---|---|---|---|---|---|---|---|
| No. | salt | A-X | $R^3$ | $t_R$ (min) | method | MW (calc.) free base | $[M+H]^+$ (found) |
| 5 | HCOOH | 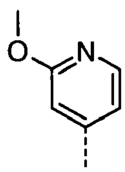 | 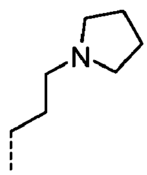 | 6.93 | A | 559.79 | 560 |
| 6 | HCOOH | 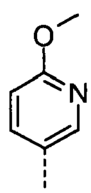 | 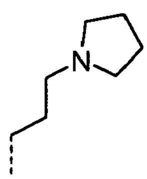 | 6.63 | A | 559.79 | 560 |

**Table 3**

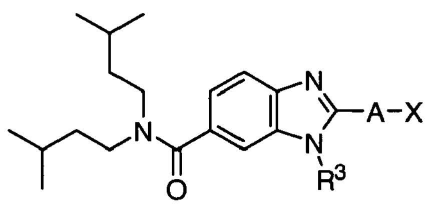

| | | | | HPLC | | MS | |
|---|---|---|---|---|---|---|---|
| No. | salt | A-X | $R^3$ | $t_R$ (min) | method | MW (calc.) free base | $[M+H]^+$ (found) |
| 7 | - | 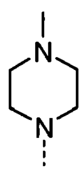 | 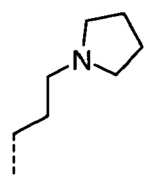 | 6.11 | A | 510.77 | 511 |

(continued)

| | | | | HPLC | | MS | |
|---|---|---|---|---|---|---|---|
| No. | salt | A-X | $R^3$ | $t_R$ (min) | method | MW (calc.) free base | $[M+H]^+$ (found) |
| 8 | 2x HCOOH | 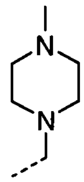 | 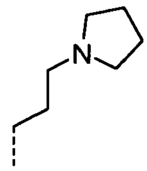 | 3.56 | A | 524.79 | 525 |
| 9 | citrate | 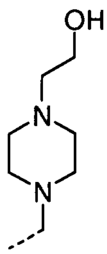 | 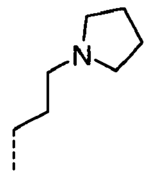 | 4.00 | A | 554.82 | 555 |
| 10 | 3x HCOOH | 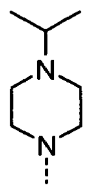 | 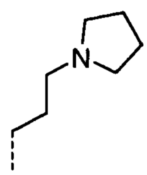 | 4.54 | A | 552.85 | 553 |

**Table 4**

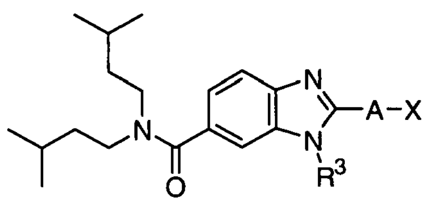

| | | | | HPLC | | MS | |
|---|---|---|---|---|---|---|---|
| No. | salt | A-X | $R^3$ | $t_R$ (min) | method | MW (calc.) free base | $[M+H]^+$ (found) |
| 11 | - | 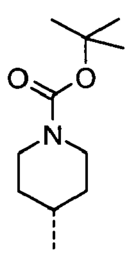 | 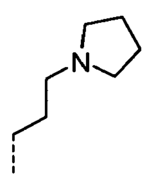 | 7.27 | A | 595.87 | 597 |

(continued)

| | | | | HPLC | | MS | |
|---|---|---|---|---|---|---|---|
| No. | salt | A-X | $R^3$ | $t_R$ (min) | method | MW (calc.) free base | $[M+H]^+$ (found) |
| 12 | 2x HCOOH | 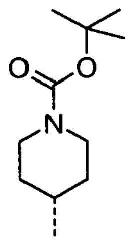 | 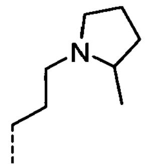 | 6.87 | A | 609.89 | 611 |
| 13 | - | 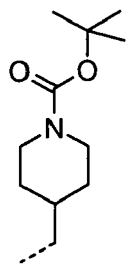 | 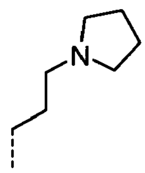 | 6.06 | A | 609.89 | 610 |
| 14 | 4x HCOOH | 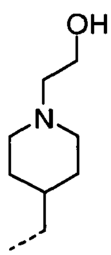 | 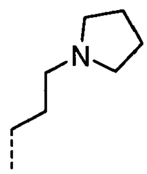 | 3.78 | A | 553.83 | 554 |
| 15 | citrate | 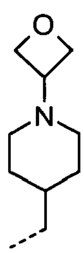 | 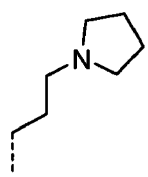 | 4.10 | A | 565.84 | 566 |
| 16 | - | 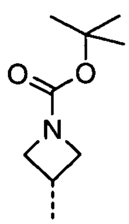 | 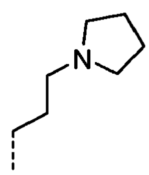 | 7.53 | A | 567.81 | 568 |
| 17 | - | 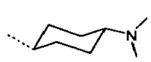 | 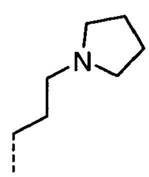 | 2.69 | B | 537.83 | 538 |

(continued)

| | | | | HPLC | | MS | |
|---|---|---|---|---|---|---|---|
| No. | salt | A-X | $R^3$ | $t_R$ (min) | method | MW (calc.) free base | $[M+H]^+$ (found) |
| 18 | 3x HCOOH | 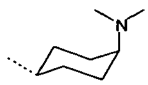 | 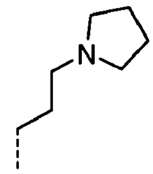 | 2.90 | B | 537.83 | 538 |

**Table 5**

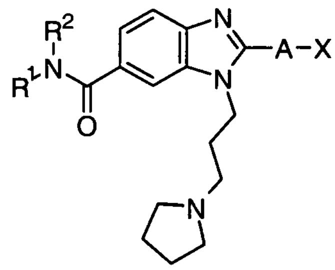

| | | | | HPLC | | MS | |
|---|---|---|---|---|---|---|---|
| No. | salt | A-X | $NR^1R^2$ | $t_R$ (min) | method | MW (calc.) free base | $[M+H]^+$ (found) |
| 19 | 2x HCOOH | 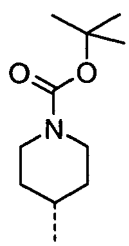 | 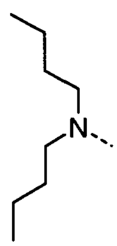 | 3.96 | B | 581.84 | 583 |
| 20 | 3x HCOOH | 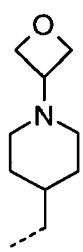 | 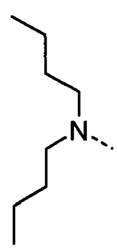 | 4.80 | A | 537.79 | 538 |

**Table 6**

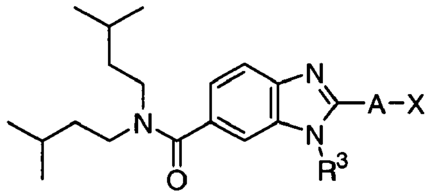

| | | | | HPLC | | MS | |
|---|---|---|---|---|---|---|---|
| No. | salt | A-X | $R^3$ | $t_R$ (min) | method | MW (calc.) free base | $[M+H]^+$ (found) |
| 21 | 2xHCl | 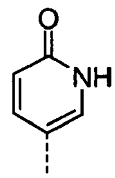 | 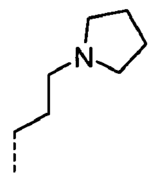 | 5.12 | A | 505.70 | 506 |
| 22 | 2xHCl | 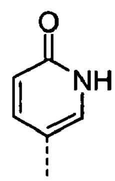 | 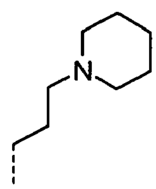 | 5.40 | A | 519.73 | 520 |
| 23 | 3 x HCl | 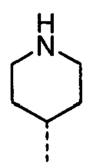 | 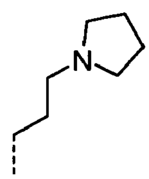 | 4.14 | A | 495.75 | 496 |
| 24 | 3 x HCl | 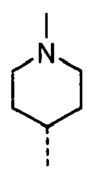 | 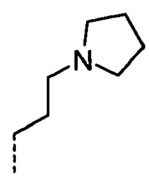 | 4.15 | A | 509.78 | 510 |
| 25 | 2xHCl | 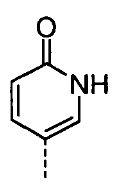 | 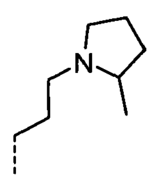 | 5.27 | A | 519.73 | 520 |
| 26 | 3 x HCl | 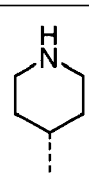 | 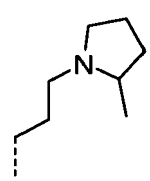 | 2.79 | C | 509.78 | 510 |

(continued)

| | | | | HPLC | | MS | |
|---|---|---|---|---|---|---|---|
| No. | salt | A-X | $R^3$ | $t_R$ (min) | method | MW (calc.) free base | $[M+H]^+$ (found) |
| 27 | 2x HCOOH | 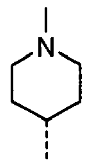 | 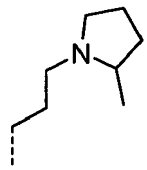 | 4.36 | A | 523.81 | 524 |
| 28 | 3 x HCl | 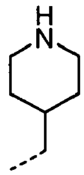 | 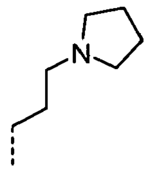 | 3.75 | A | 509.78 | 510 |
| 29 | 2x HCOOH | 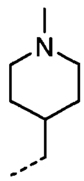 | 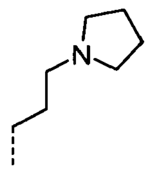 | 4.09 | A | 523.81 | 524 |
| 30 | 2 x HCl | 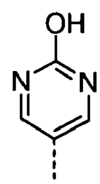 | 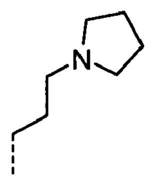 | 5.18 | A | 506.69 | 507 |
| 31 | 3 x HCl | 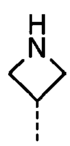 | 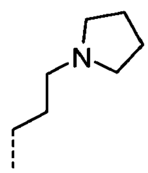 | 4.19 | A | 467.70 | 468 |
| 32 | 2x HCOOH | 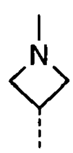 | 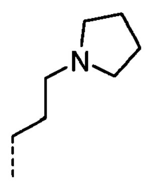 | 4.56 | A | 481.72 | 482 |
| 33 | HCOOH | 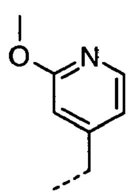 | 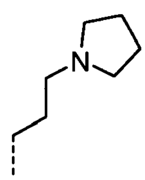 | 6.23 | A | 533.76 | 534 |

(continued)

| | | | | HPLC | | MS | |
|---|---|---|---|---|---|---|---|
| No. | salt | A-X | $R^3$ | $t_R$ (min) | method | MW (calc.) free base | $[M+H]^+$ (found) |
| 34 | HCOOH | 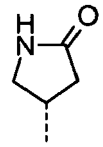 | 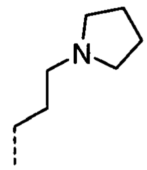 | 5.34 | A | 495.71 | 497 |
| 35 | HCOOH | 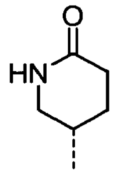 | 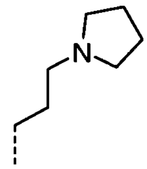 | 5.61 | A | 509.734 | 510 |
| 36 | HCOOH | 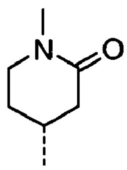 | 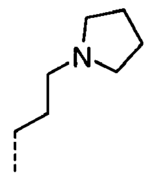 | 5.47 | A | 523.76 | 524 |
| 37 | HCOOH | 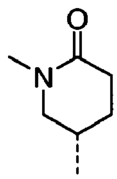 | 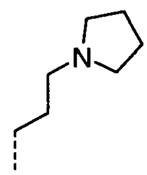 | 5.94 | A | 523.76 | 524 |

**Table 7**

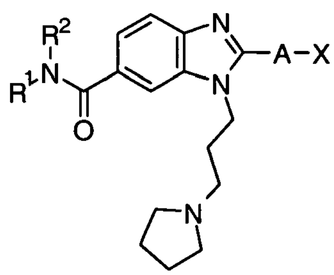

(continued)

| | | | | HPLC | | MS | |
|---|---|---|---|---|---|---|---|
| No. | salt | A-X | $NR^1R^2$ | $t_R$ (min) | method | MW (calc.) free base | $[M+H]^+$ (found) |
| 38 | 3 x HCl | 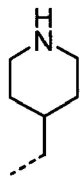 | 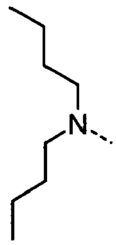 | 2.96 | A | 481.72 | 482 |
| 39 | 3x HCOOH | 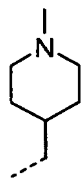 | 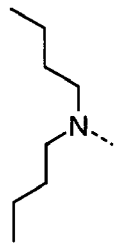 | 4.41 | A | 495.75 | 496 |

The following examples are provided to illustrate the invention and are not limiting the scope of the invention in any manner.

**Synthesis of Example 1:**

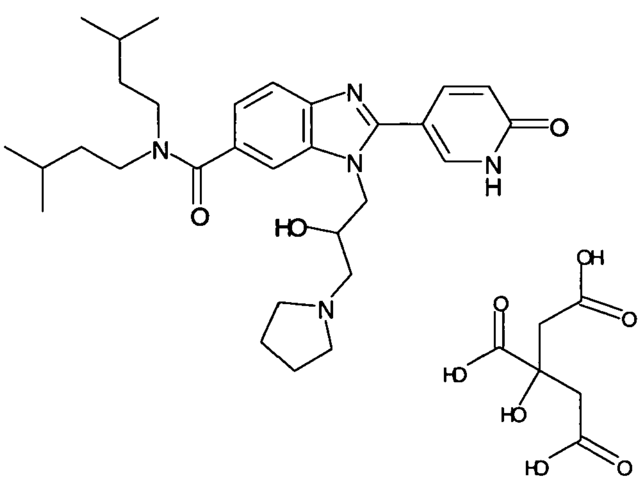

At 0˚C, under argon, to a stirring solution of example 3 (47.1 mg) in 7.5 mL dry DCM were added dropwise 225 μL of a 1 M solution of $BBr_3$ in DCM. The reaction mixture was slowly allowed to warm to room temperature and stirred for 2 days. LCMS showed complete conversion. The reaction mixture was poured in saturated sodium bicarbonate solution (50 mL) and the aqueous layer was extracted with DCM (3x25 mL). The combined organic layer was washed with brine, dried over sodium sulfate and the solvent was removed under reduced pressure. The obtained transparent solid was dissolved in EtOAc (6 mL) and EtOH (750 μL). To this solution was added a 0.1N citric acid solution in EtOH (750 μL) and the reaction mixture was stirred at room temperature for 2h. Volatiles were removed under reduced pressure and the obtained solid was dissolved in water (3 mL) and lyophilized.

**Synthesis of Example 2:**

***Intermediate 2a):***

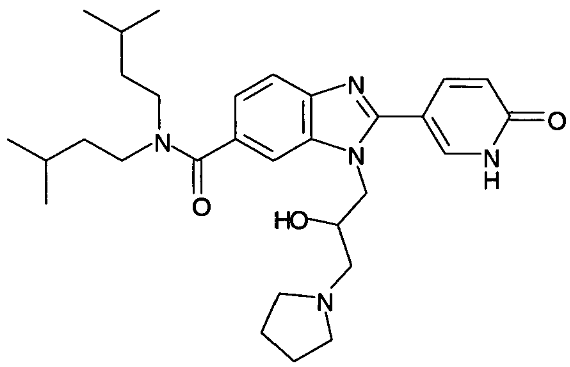

To a solution of intermediate 3d (442 mg, 1.06 mmol) in DMF (20 mL) was added 6-hydroxynicotinaldehyde (130 mg, 1.06 mmol). The reaction mixture was stirred vigorously at 90°C for 24 h. Additional 6-hydroxynicotinaldehyde (123 mg, 1.00 mmol) was added and stirring at reflux temperature was continued overnight. The solvent was removed under reduced pressure. The residue was taken up in ethyl acetate and the resulting solution was extracted twice with saturated sodium bicarbonate solution. The combined organic layer was extracted twice with ethyl acetate. The combined organic layer was washed with brine, dried over sodium sulfate and the solvent was removed under reduced pressure.
The crude product was purified by preparative LC-MS.

***Example 2:***

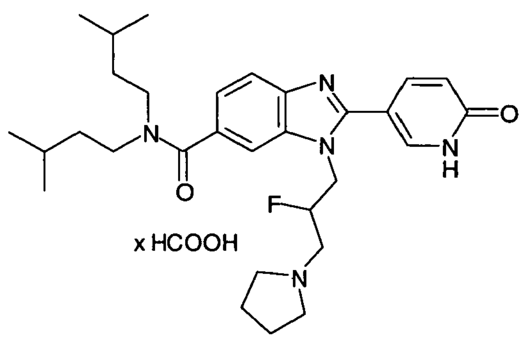

x HCOOH

Intermediate 2a (~80% purity, 217 mg, formate salt) was dissolved in ethyl acetate and washed twice with saturated sodium bicarbonate solution. The combined aqueous layer was extracted with ethyl acetate and the combined organic layer was washed with brine and dried over sodium sulfate. The solvent was removed under reduced pressure. The free base (148 mg, 0.284 mmol) was dissolved in dry toluene (10 mL) and dry pyridine (1.5 mL) under argon atmosphere. Bis(2-methoxyethyl)aminosulfur trifluoride (503 mg, 2.272 mmol) was added dropwise and the reaction mixture was stirred at room temperature for 30 min. The reaction mixture was diluted with ethyl acetate (30 mL) and hydrolyzed by addition of 10% aqueous sodium carbonate solution. The organic phase was separated and the aqueous layer extracted twice with ethyl acetate. The combined organic layer was washed with water and brine, dried over sodium sulfate and the solvent was removed under reduced pressure.
The crude product was purified by preparative LC-MS.

**Synthesis of Example 3:**

***Intermediate 3a):***

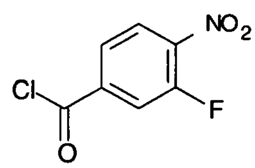

At 0˚C to a solution of 3-fluoro-4-nitrobenzoic acid (2.22 g, 12.0 mmol) in dry DCM (150 mL) under argon atmosphere was added oxalyl chloride (1117 μL, 13.2 mmol) dropwise. DMF (2-3 drops) was added and the reaction mixture was stirred at 0˚C for 1 h and at room temperature for 2 h. Volatiles were removed under reduced pressure and the residue was twice co-evaporated with toluene.

The product was used as such for the next step without further purification.

***Intermediate 3b):***

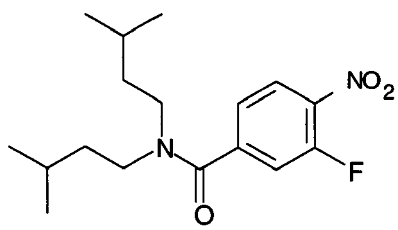

At 0˚C to a solution of intermediate 3a (12.0 mmol) in dry DCM (100 mL) under argon atmosphere was added diisoamylamine (2.58 mL, 12.6 mmol) and DIEA (2.19 mL, 12.6 mmol) dropwise and the reaction mixture was stirred at 0˚C for 1 h and at room temperature overnight. The reaction mixture was poured into water (100 mL) and the organic layer was separated. The aqueous layer was extracted twice with DCM. The combined organic layers were washed three times with 1 N aqueous HCl solution and three times with saturated aqueous sodium bicarbonate solution, water, brine, dried over sodium sulfate and the solvent was removed under reduced pressure.

***Intermediate 3c):***

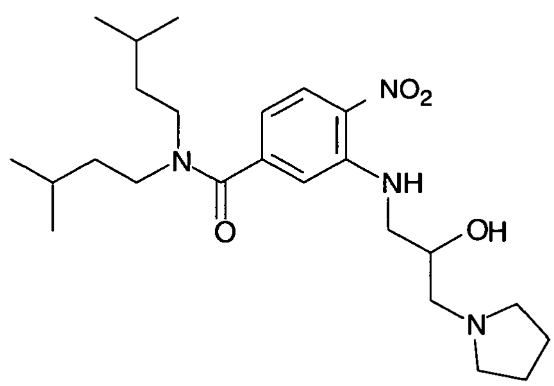

To 3-fluoro-N,N-bis-(3-methyl-butyl)-4-nitro-benzamide (880 mg, 2.71 mmol) and potassium carbonate (749 mg, 5.42 mmol) in acetonitrile (40 mL) was added 1-amino-3-pyrrolidin-1-yl-propan-2-ol (469 mg, 3.25 mmol). The mixture was kept under reflux for 4 h and then concentrated at reduced pressure. The residue was taken up with DCM (60 mL) and water (30 mL). The aqueous phase was extracted twice with DCM and the combined organic fractions were washed with water and brine, dried over sodium sulfate and the solvent was removed under reduced pressure.

***Intermediate 3d):***

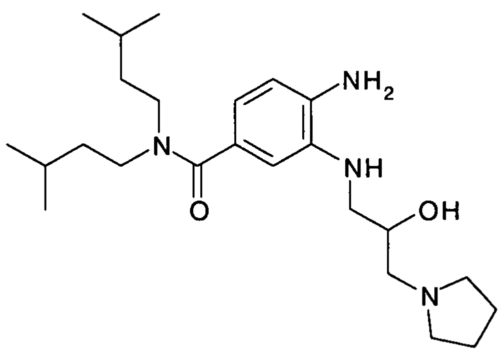

A mixture of intermediate 3c (650 mg, 1.45 mmol) and 10% palladium on charcoal (309 mg, 0.29 mmol) in ethanol (25 mL) and ethyl acetate (25 mL) was evacuated and purged with argon three times and then evacuated and flushed with hydrogen three times. The reaction mixture was stirred at room temperature under atmospheric pressure of hydrogen for 30 min. The reaction mixture was filtered through Celite and the filter cake was washed with ethanol. The filtrate was evaporated.

The crude product was used for the next step without purification.

***Intermediate 3e):***

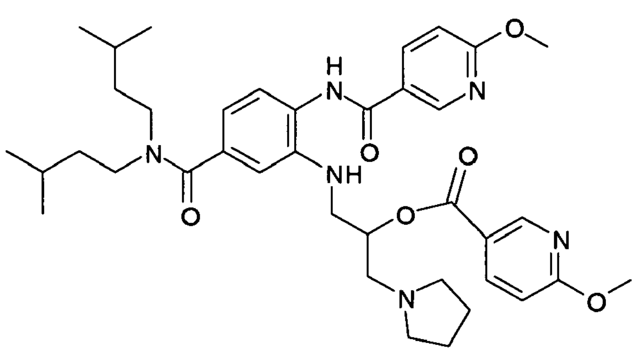

To a solution of intermediate 3d (145 mg, 0.346 mmol) in DCM (10 mL) was added 2-methoxy-5-pyridinecarboxylic acid (56 mg, 0.363 mmol), EDC (70 mg, 0.363 mmol) and DMAP (5 mg) and the solution was stirred at room temperature for 18 h. LC-MS showed still some starting material. Therefore, additional 2-methoxy-5-pyridinecarboxylic acid (56 mg, 0.363 mmol), EDC (70 mg, 0.363 mmol) and DMAP (5 mg) were added and stirring was continued for 3 d. The reaction mixture was diluted with DCM (50 ml) and extracted twice with water, saturated sodium bicarbonate solution and with brine. The organic layer was dried over sodium sulfate and the solvent was removed under reduced pressure.

The crude product (mixture of mono- and diacylated product) was used as such for the next step.

***Intermediate 3f):***

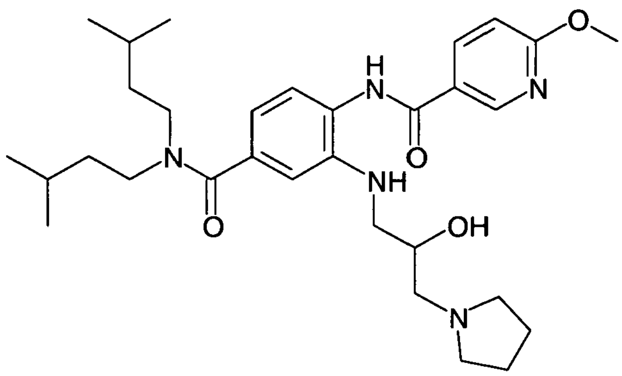

Intermediate 3e (0.346 mmol) was dissolved in THF (5 mL) and cooled to 0˚C. Lithium hydroxide monohydrate (44 mg, 1.038 mmol) in water (1 mL) was slowly added and the reaction mixture was stirred at 0˚C for 30 min and at room temperature for 30 h. The reaction mixture was concentrated and then partitioned between ethyl acetate and saturated sodium bicarbonate solution. The aqueous layer was extracted twice with ethyl acetate. The combined organic layer was washed with brine, dried over sodium sulfate and the solvent was removed under reduced pressure.

The crude product was purified by column chromatography.

***Example 3:***

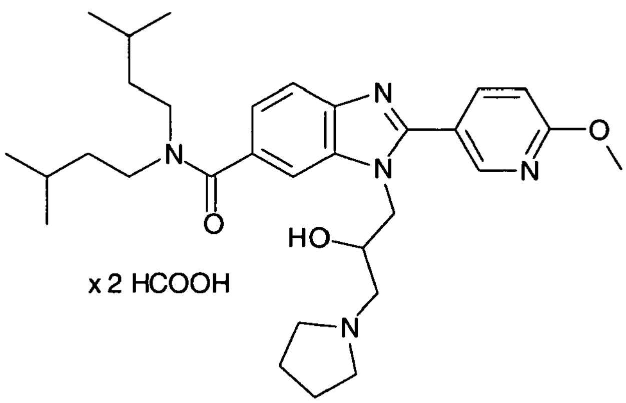

Intermediate 3f (89 mg, 0.161 mmol) was dissolved in acetic acid (5 mL) and stirred at 80˚C for 23 h. The solvent was removed under reduced pressure.

The crude product was purified by preparative LC-MS.

**Synthesis of Example 4:**

***Intermediate 4a):***

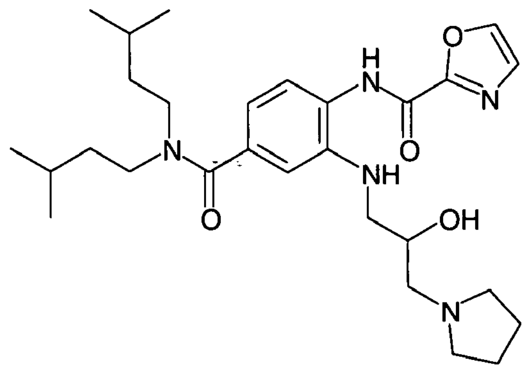

To a solution of intermediate 3d (7.07 g, 16.9 mmol) in DCM (200 mL) was added oxazole-2-carboxylic acid (4.00 g, 35.4 mmol), EDC (6.79 g, 35.4 mmol) and DMAP (200 mg, 1.69 mmol) and the suspension was stirred at room temperature for 22 h. The reaction mixture was diluted with DCM (300 mL) and extracted twice with water, saturated sodium bicarbonate solution and with brine. The organic layer was dried over sodium sulfate and the solvent was removed under reduced pressure.

The crude product was purified by column chromatography.

***Example 4:***

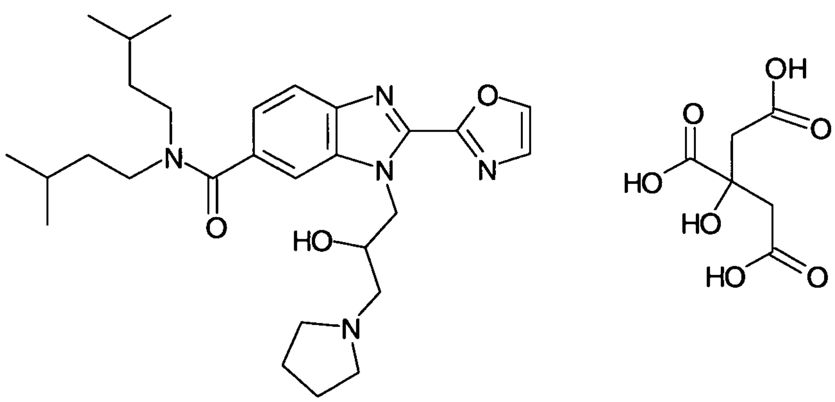

Intermediate 4a (1.77 g) was dissolved in acetic acid (50 mL) and stirred at 90˚C for 3 h. The reaction mixture was concentrated and then partitioned between ethyl acetate and saturated sodium bicarbonate solution. The organic layer was washed with saturated sodium bicarbonate solution. The combined aqueous layer was extracted twice with ethyl acetate. The combined organic layer was washed with water and brine, dried over sodium sulfate and the solvent was removed under reduced pressure. The crude product was purified by column chromatography.

The pure product (758 mg, 1.53 mmol) was dissolved in MeOH (10 mL) and 0.1 M citric acid in ethanol (15.3 mL, 1.53 mmol) was added. The solution was filtered and solvents were removed under reduced pressure. The product was taken up in water (20 mL) and lyophilized.

**Synthesis of Example 5:**

***Intermediate 5a):***

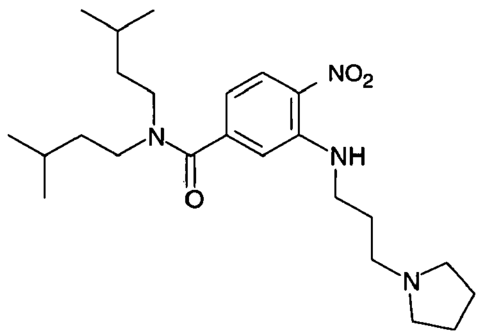

To 3-fluoro-N,N-bis-(3-methyl-butyl)-4-nitro-benzamide (16.35 g, 50.4 mmol) and potassium carbonate (13.93 g, 100.8 mmol) in acetonitrile (600 ml) was added 1-(3-aminopropyl)pyrrolidine (7.01 mL, 55.4 mmol). The mixture was kept under reflux for 3.5 h and then concentrated at reduced pressure. The residue was taken up with DCM (800 mL) and water (400 mL). The aqueous phase was extracted twice with DCM and the combined organic fractions were washed with water and brine, dried over sodium sulfate and the solvent was removed under reduced pressure.

***Intermediate 5b):***

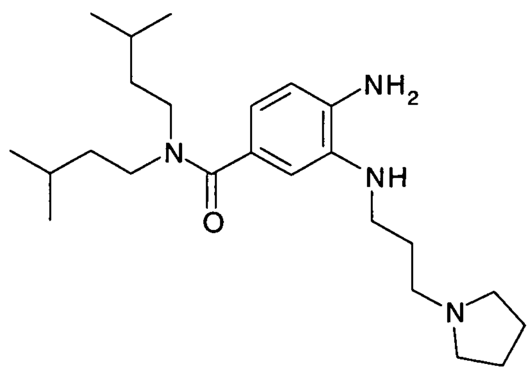

A mixture of intermediate 5a (50.9 mmol) and 10% palladium on charcoal (10.9 g, 10.2 mmol) in ethanol (300 mL) and ethyl acetate (300 mL) was evacuated and purged with argon three times and then evacuated and flushed with hydrogen three times. The reaction mixture was stirred at room temperature under atmospheric pressure of hydrogen for 2.5 h. The reaction mixture was filtered through Celite and the filter cake was washed with ethanol. The filtrate was evaporated.

The crude product was used for the next step without purification.

***Intermediate 5c):***

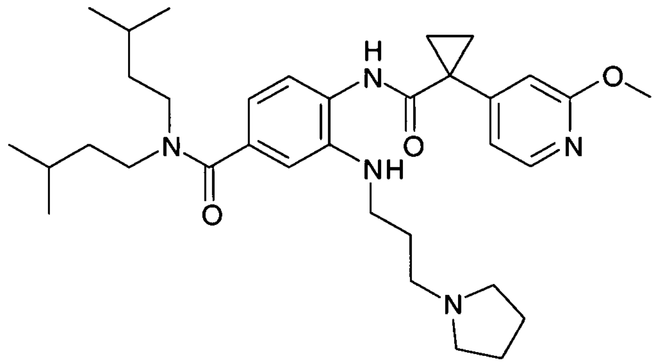

To a solution of intermediate 5b (201 mg, 0.500 mmol) in DCM (10 mL) was added 1-(2-methoxypyridin-4-yl) cyclopropanecarboxylic acid (101 mg, 0.525 mmol), EDC (101 mg, 0.525 mmol) and DMAP (5 mg) and the solution was stirred at room temperature for 4 d. LC-MS indicated only partial conversion. The reaction mixture was diluted with DCM (50 mL) and extracted twice with water, saturated sodium bicarbonate solution and with brine. The organic layer was dried over sodium sulfate and the solvent was removed under reduced pressure.

The crude product was purified by column chromatography.

***Example 5:***

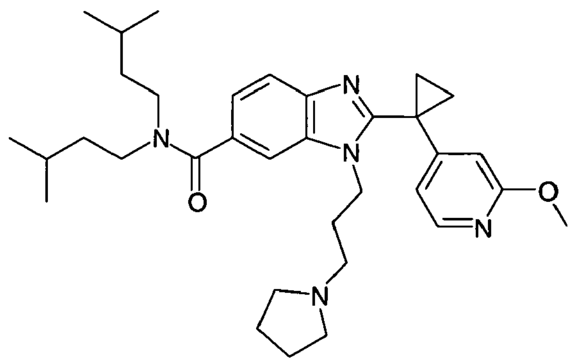

Intermediate 5c (44 mg, 0.076 mmol) was dissolved in acetic acid (5 mL) and stirred at 80˚C for 24 h. The solvent was removed under reduced pressure.

The crude product was purified by preparative LC-MS.

**Synthesis of Example 6:**

***Intermediate 6a):***

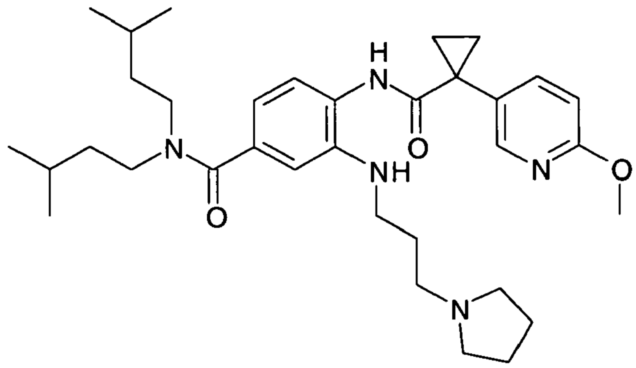

To a solution of intermediate 5b (201 mg, 0.500 mmol) in DCM (10 ml) was added 1-(6-methoxypyridin-3-yl) cyclopropanecarboxylic acid (101 mg, 0.525 mmol), EDC (101 mg, 0.525 mmol) and DMAP (5 mg) and the solution was stirred at room temperature for 4 d. LC-MS indicated only partial conversion. The reaction mixture was diluted with DCM (50 mL) and extracted twice with water, saturated sodium bicarbonate solution and with brine. The organic layer was dried over sodium sulfate and the solvent was removed under reduced pressure.

The crude product was purified by column chromatography.

***Example 6:***

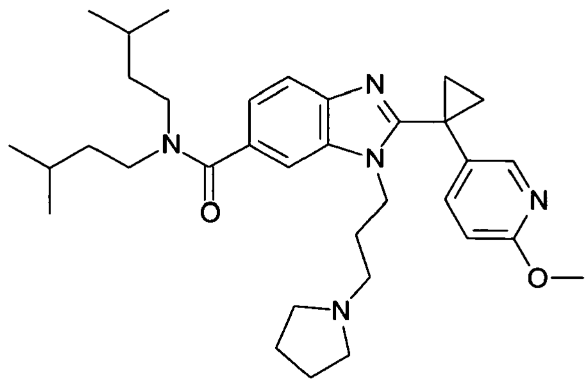

Intermediate 6a (41 mg, 0.071 mmol) was dissolved in acetic acid (5 mL) and stirred at 80˚C for 19 h. The solvent was removed under reduced pressure.

The crude product was purified by preparative LC-MS.

**Synthesis of Example 7:**

***Intermediate 7a):***

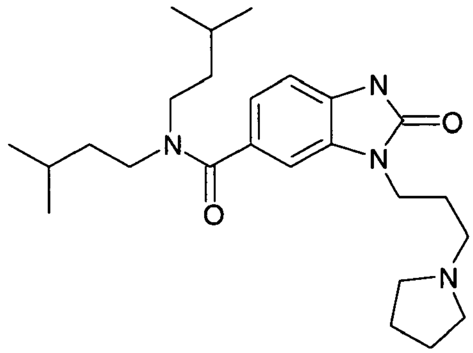

To a solution of intermediate 5b (500 mg) in 20 mL of dioxane were added 100 μL of pyridine and 122 mg of triphosgene dissolved in 5 mL of dioxane. The reaction mixture was stirred at room temperature over night and was then simply extensively evaporated to yield a crude brownish syrup. It was then taken back in DCM and was washed with $NH_4Cl$ (0.2 M). The aqueous phase was extracted back with DCM and an emulsion appeared. The latter was extracted with AcOEt. DCM phase and AcOEt phase were washed a last time with brine/NaOH (0.1 M), merged, dried over $Na_2SO_4$ and partially evaporated. In order to form the salt 2 mL of HCl 1 M in $Et_2O$ was added, then the solvent was extensively evaporated. The crude product was used in the next step without further purification.

***Intermediate 7b):***

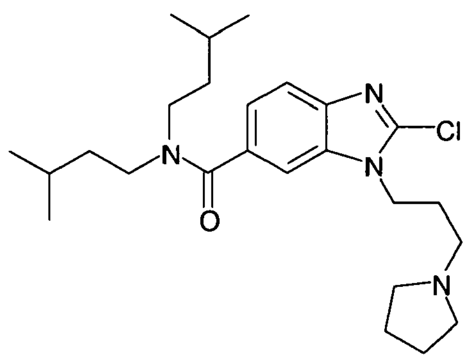

Intermediate 7a (1.4 mmol) was dissolved in 4 ml of phosphorus (V) oxychloride reagent under Ar. The resulting dark solution was then heated up to refluxing conditions (oil bath at 120 °C) for 2h30. Then phosphorus (V) oxychloride was removed under high vacuum and the crude product was used in the next step without further purification.

***Example 7:***

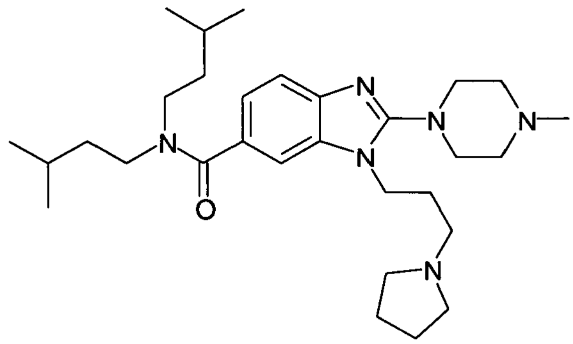

Intermediate 7b (220 mg) was dissolved in about 2 mL of 1-methylpiperazine. The reaction mixture was left running for 2 days at room temperature and at 45 ˚C for 5h for achieving complete conversion. The solvent was removed under reduced pressure.

The crude product was purified by preparative LC-MS.

**Synthesis of Example 8:**

***Intermediate 8a):***

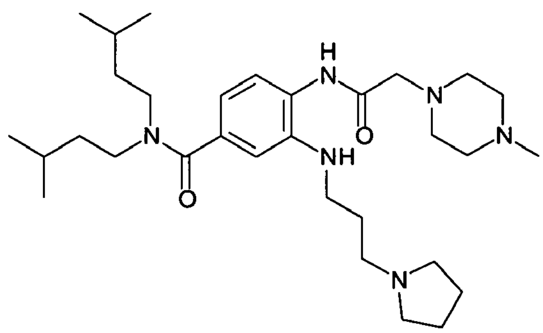

To a solution of intermediate 5b (201 mg, 0.500 mmol) in DCM (10 mL) was added (4-methylpiperazin-1-yl)-acetic acid (83 mg, 0.525 mmol), EDC (101 mg, 0.525 mmol) and DMAP (5 mg) and the solution was stirred at room temperature for 15 h. LC-MS indicated only partial conversion. Therefore, stirring was continued for 7 h. No more conversion took place. NMM (58 mL, 0.525 mmol) was added and stirring was continued for 3 d. The reaction mixture was diluted with DCM (50 mL) and extracted twice with water, saturated sodium bicarbonate solution and with brine. The organic layer was dried over sodium sulfate and the solvent was removed under reduced pressure.

The crude product was purified by column chromatography.

***Example 8:***

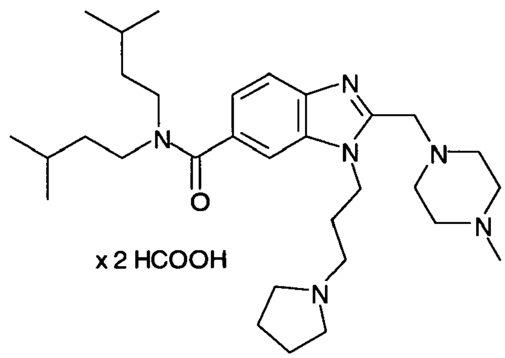

x 2 HCOOH

Intermediate 8a (88 mg, 0.162 mmol) was dissolved in acetic acid (5 mL) and stirred at 80˚C for 6.5 h. The solvent was removed under reduced pressure.

The crude product was purified by preparative LC-MS.

**Synthesis of Example 9:**

***Intermediate 9a):***

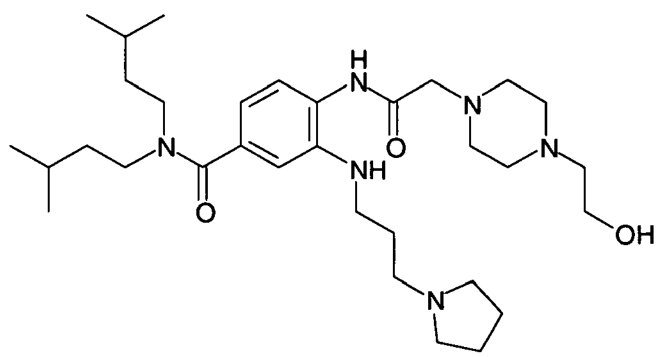

To a solution of intermediate 5b (1832 mg, 4.55 mmol) in DCM (100 mL) was added [4-(2-hydroxyethyl)-piperazin-1-yl]-acetic acid (900 mg, 4.78 mmol), EDC (916 mg, 4.78 mmol), DMAP (50 mg) and NMM (525 μL, 4.78 mmol) and the solution was stirred at room temperature for 17 h. The reaction mixture was diluted with DCM (500 mL) and extracted twice with water, saturated sodium bicarbonate solution and with brine. The organic layer was dried over sodium sulfate and the solvent was removed under reduced pressure.

The crude product was purified by column chromatography.

***Example 9:***

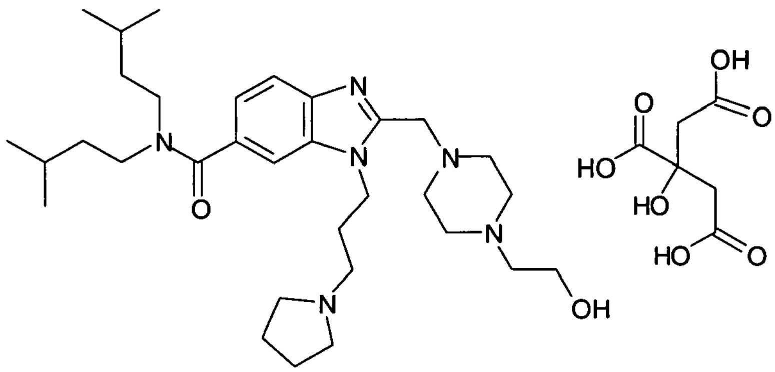

Intermediate 9a (848 mg, 1.48 mmol) was dissolved in acetic acid (40 mL) and stirred at 80˚C for 3 h. The solvent was removed under reduced pressure.

The crude product was dissolved in THF (36 mL) and cooled to 0˚C. Lithium hydroxide monohydrate (621 mg, 14.8 mmol) in water (6 mL) was slowly added and the reaction mixture was stirred at 0˚C for 30 min and at room temperature for 3 d. Additional lithium hydroxide monohydrate (621 mg, 14.8 mmol) was added and stirring was continued for 4 h. The reaction mixture was concentrated and then partitioned between ethyl acetate and water. The aqueous layer was extracted twice with ethyl acetate. The combined organic layer was washed with brine, dried over sodium sulfate and the solvent was removed under reduced pressure.

The crude product was purified by column chromatography.

The pure product (440 mg, 0.793 mmol) was dissolved in methanol (10 ml) and 0.1 M citric acid in ethanol (7.93 ml, 0.793 mmol) was added. The solution was filtered and solvents were removed under reduced pressure. The product was taken up in water (8 ml) and lyophilized.

**Synthesis of Example 10:**

***Intermediate 10a):***

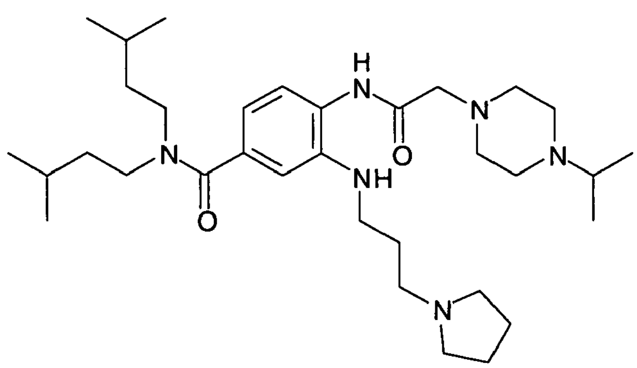

To a solution of intermediate 5b (201 mg, 0.500 mmol) in DCM (10 mL) was added (4-isopropyl-piperazin-1-yl)-acetic acid (98 mg, 0.525 mmol), EDC (101 mg, 0.525 mmol) and DMAP (5 mg) and the solution was stirred at room temperature for 15 h. LC-MS indicated only partial conversion. Therefore, stirring was continued for 7 h. No more conversion took place. NMM (58 mL, 0.525 mmol) was added and stirring was continued for 3 d. The reaction mixture was diluted with DCM (50 mL) and extracted twice with water, saturated sodium bicarbonate solution and with brine. The organic layer was dried over sodium sulfate and the solvent was removed under reduced pressure.
The crude product was purified by column chromatography.
The mixed fraction was purified by preparative LC-MS.

*Example 10:*

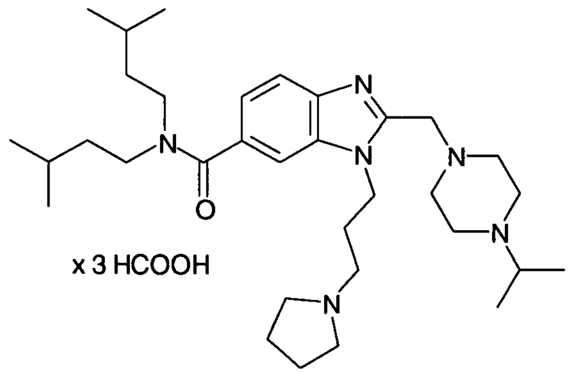

x 3 HCOOH

Intermediate 10a (0.185 mmol) was dissolved in acetic acid (5 mL) and stirred at 80°C for 4 h. The solvent was removed under reduced pressure.

The crude product was purified by preparative LC-MS.

**Synthesis of Example 11:**

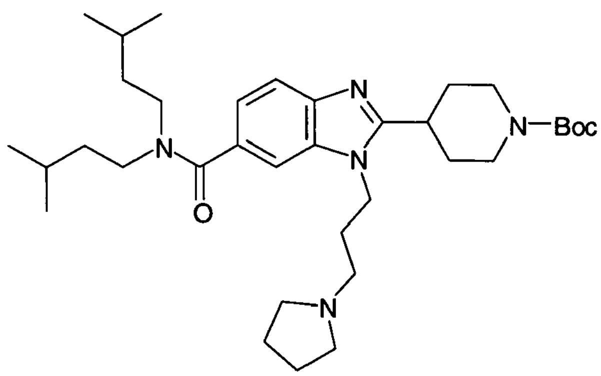

To a solution of intermediate 5b (201 mg, 0.500 mmol) in ethanol (20 mL) was added 1-Boc-4-piperidinecarboxaldehyde (107 mg, 0.500 mmol). The reaction mixture was stirred vigorously at reflux temperature for overnight. The solvent was removed under reduced pressure.

The crude product was purified by preparative LC-MS.

**Synthesis of Example 12:**

*Intermediate 12a):*

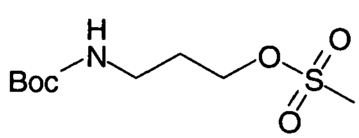

A solution of tert-butyl N-(3-hydroxypropyl)carbamate (708 μL, 5.00 mmol) and triethylamine (1045 μL, 7.50 mmol) in dry DCM (20 mL) was cooled to 0˚C under argon atmosphere. Mesyl chloride (426 μL 5.50 mmol) was added dropwise at this temperature and the reaction mixture was stirred for 2 h. The reaction mixture was diluted with DCM and washed with saturated sodium bicarbonate solution, water and brine. The aqueous layer was extracted back twice with DCM. The combined organic layer was dried over sodium sulfate and the solvent was removed under reduced pressure.

The crude product was used as such for the next step.

***Intermediate 12b):***

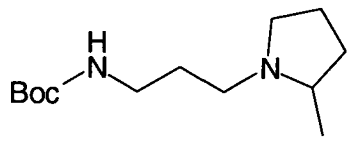

Intermediate 12a (957 mg, 3.78 mmol) was dissolved in acetonitrile (20 mL). 2-Methylpyrrolidine (1463 μL, 15.12 mmol) was added and the reaction mixture was stirred at 50˚C overnight. Volatiles were removed under reduced pressure and the residue taken up with DCM. The organic layer was washed twice with saturated sodium bicarbonate solution. The combined aqueous layer was extracted back with DCM. The combined organic layer was washed with brine, dried over sodium sulfate, and the solvent was removed under reduced pressure.

***Intermediate 12c):***

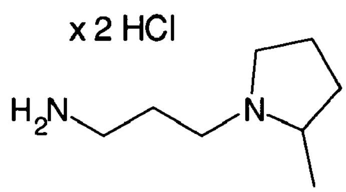

Intermediate 12b (748 mg, 3.09 mmol) was dissolved in a mixture of methanol (10 mL) and dioxane (10 mL). 4 M HCl in dioxane (10 mL) was added and the reaction mixture was stirred for 1 h. Solvents were removed under reduced pressure.

The crude product was used as such for the next step.

***Intermediate 12d):***

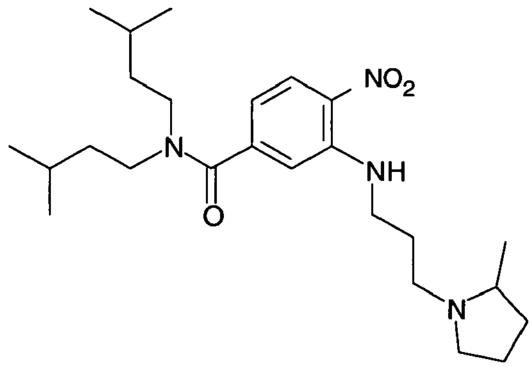

To 3-fluoro-N,N-bis-(3-methyl-butyl)-4-nitro-benzamide (830 mg, 2.56 mmol) and potassium carbonate (1769 mg, 12.8 mmol) in acetonitrile (50 mL) was added intermediate 12c (3.09 mmol). The mixture was kept under reflux for 3 d and then concentrated at reduced pressure. The residue was taken up with DCM (60 mL) and water (30 mL). The aqueous phase was extracted twice with DCM and the combined organic fractions were washed with water and brine, dried over sodium sulfate and the solvent was removed under reduced pressure.

***Intermediate 12e):***

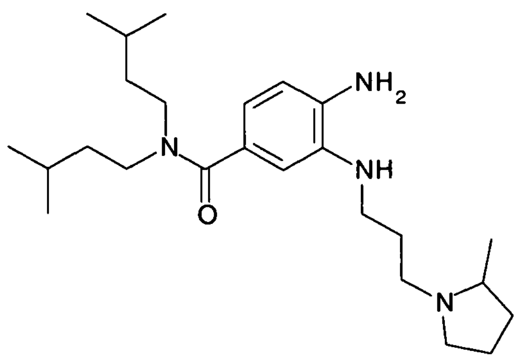

A mixture of intermediate 12d (1108 mg, 2.48 mmol) and 10% palladium on charcoal (528 mg, 0.496 mmol) in ethanol (30 ml) and ethyl acetate (30 mL) was evacuated and purged with argon three times and then evacuated and flushed with hydrogen three times. The reaction mixture was stirred at room temperature under atmospheric pressure of hydrogen for 3.5 h. The reaction mixture was filtered through Celite and the filter cake was washed with ethanol. The filtrate was evaporated.

The crude product was used for the next step without purification.

***Example 12:***

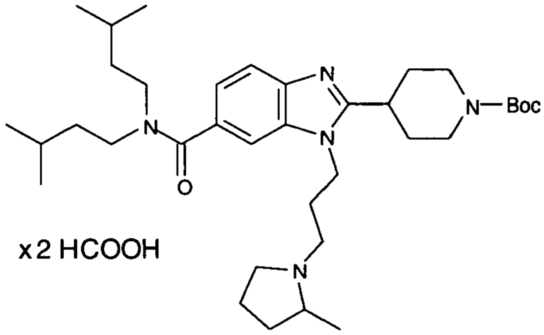

x2 HCOOH

To a solution of intermediate 12e (570 mg, 1.37 mmol) in ethanol (30 mL) was added 1-Boc-4-piperidinecarboxaldehyde (292 mg, 1.37 mmol). The reaction mixture was stirred vigorously at reflux temperature for overnight. The solvent was removed under reduced pressure.

The crude product was purified by preparative LC-MS.

**Synthesis of Example 13:**

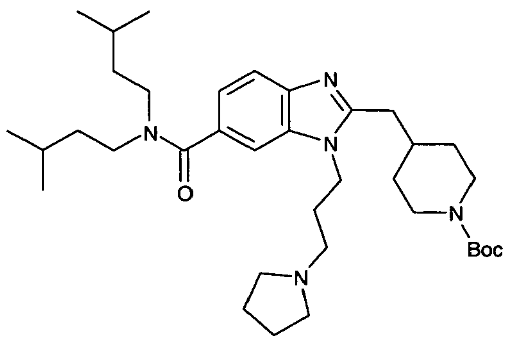

To a solution of intermediate 5b (467 mg, 1.16 mmol) in ethanol (30 mL) was added N-Boc-4-piperidineacetaldehyde (264 mg, 1.16 mmol). The reaction mixture was stirred vigorously at reflux temperature for 36 h. The solvent was removed under reduced pressure.

The crude product was purified by preparative LC-MS.

**Synthesis of Example 14:**

***Intermediate 14a):***

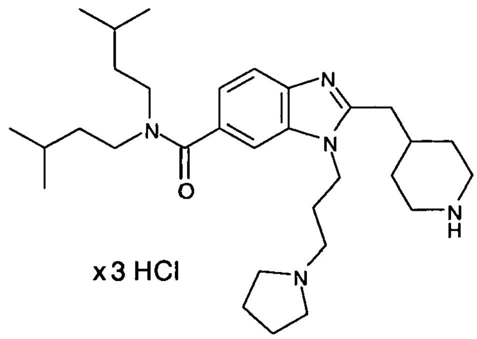

x 3 HCl

To a solution of example 13 (426 mg, 0.70 mmol) in dioxane (5 mL) was added 4 N HCl in dioxane (15 mL) and methanol (1 mL). The reaction mixture was stirred at room temperature for 45 min. Volatiles were removed and the residue was co-evaporated twice with toluene (10 mL). The product was dried under reduced pressure.

***Example 14:***

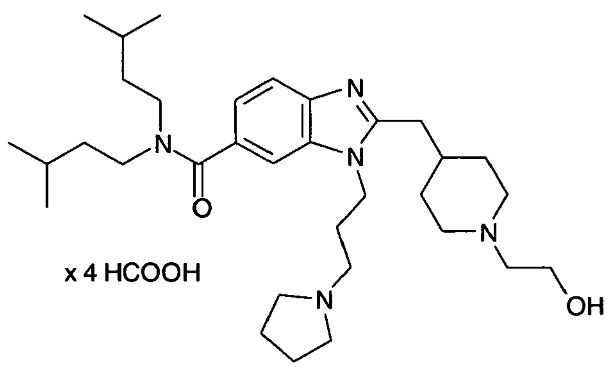

x 4 HCOOH

To a suspension of intermediate 14a (161 mg, 0.260 mmol) in acetonitrile (5 mL) was added potassium carbonate (287 mg, 2.08 mmol) followed by 2-bromoethanol (22 μL, 0.312 mmol) and the reaction mixture was stirred at reflux temperature for 5 h. Volatiles were removed and the residue was divided between ethyl acetate (60 mL) and saturated aqueous sodium carbonate solution (20 mL). The organic layer was washed twice with saturated aqueous sodium carbonate solution (20 mL each). The aqueous layer was extracted back with ethyl acetate (20 mL). The combined organic layer was washed with brine, dried over sodium sulfate and the solvent was removed under reduced pressure.

The crude product was purified by preparative LC-MS.

**Synthesis of Example 15:**

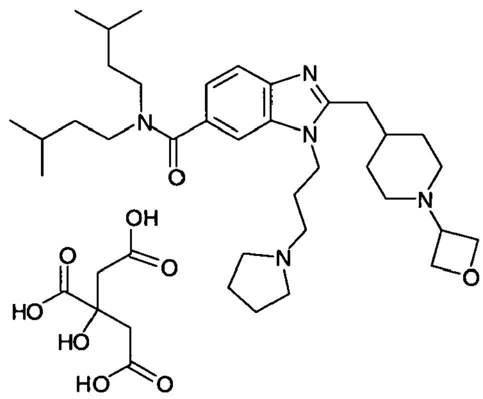

To a suspension of example 28 (136 mg, 0.220 mmol) in 1,2-dichloroethane (5 mL) was added triethylamine (92 μL, 0.660 mmol). The reaction mixture was stirred for 5 min, then 3-oxetanone (19 mg, 0.264 mmol) was added and stirring was continued for 5 min. Sodium triacetoxyborohydride (56 mg, 0.264 mmol) was added and the reaction mixture was stirred overnight. Volatiles were removed and the residue was divided between ethyl acetate (60 mL) and saturated aqueous sodium carbonate solution (20 mL). The organic layer was washed twice with saturated aqueous sodium carbonate solution (20 ml each). The aqueous layer was extracted back with ethyl acetate (20 mL). The combined organic layer was washed with brine, dried over sodium sulfate and the solvent was removed under reduced pressure.

The crude product was purified by preparative LC-MS.

The pure product (54.7 mg, 0.0967 mmol) was dissolved in MeOH (1 mL) and 0.1 M citric acid in ethanol (967 μL, 0.0967 mmol) was added. The solvents were removed under reduced pressure. The product was taken up in water (4 mL) and lyophilized.

**Synthesis of Example 16:**

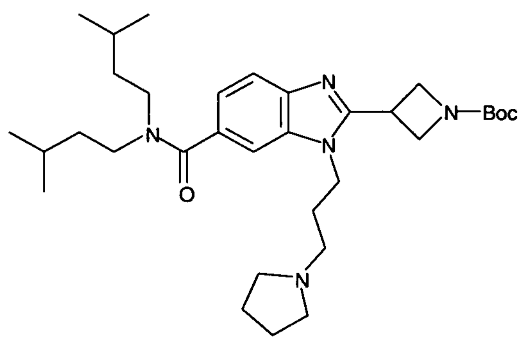

To a solution of intermediate 5b (380 mg, 0.94 mmol) in ethanol (30 mL) was added 1-Boc-3-azetidinecarboxaldehyde (174 mg, 0.94 mmol). The reaction mixture was stirred vigorously at reflux temperature overnight. The solvent was removed under reduced pressure.

The crude product was purified by preparative LC-MS.

**Synthesis of Example 17:**

***Intermediate 17a):***

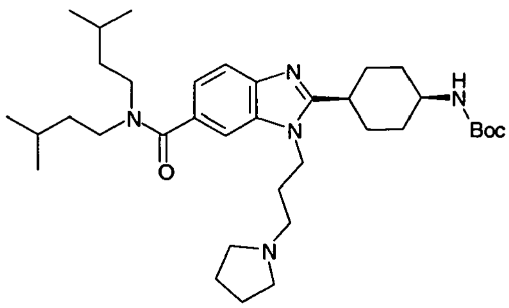

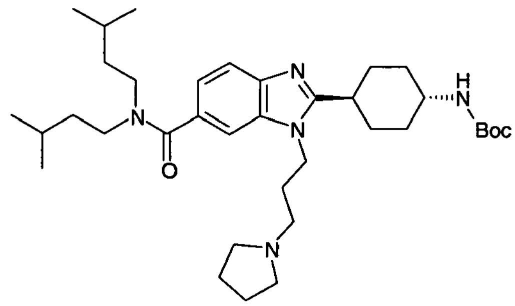

To a solution of intermediate 5b (1007 mg, 2.50 mmol) in ethanol (50 mL) was added cis-4-(tert-butoxycarbonylamino)-cyclohexane-carbaldehyde (568 mg, 2.50 mmol). The reaction mixture was stirred vigorously at reflux temperature overnight. The solvent was removed under reduced pressure.

The crude product was purified by preparative LC-MS and intermediate 17a cis and 17a trans were obtained.

***Intermediate 17b):***

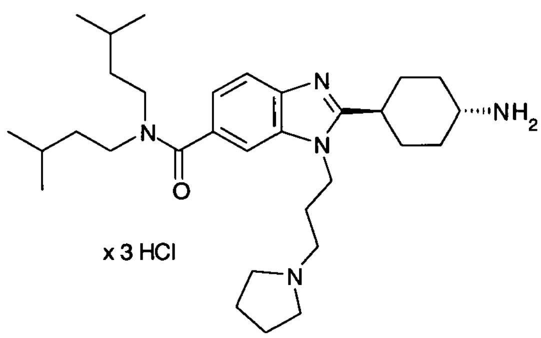

x 3 HCl

To a solution of product from intermediate 17a trans (113 mg, 0.185 mmol) in dioxane (2 mL) was added 4 N HCl in dioxane (6 mL) and methanol (1 mL). The reaction mixture was stirred at room temperature for 90 min. Volatiles were removed and the residue was co-evaporated twice with toluene (5 mL). The product was dried under reduced pressure.

***Example 17:***

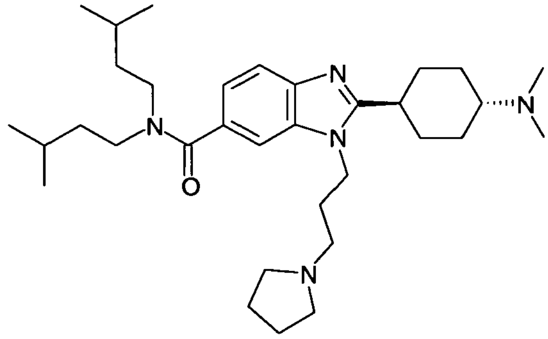

To a solution of intermediate 17b (100 mg, 0.162 mmol) in formic acid (2750 μL, 72.9 mmol) was added formaldehyde (36.5 % aq., 3099 μL, 40.5 mmol). The reaction mixture was stirred at 100°C overnight. Volatiles were removed and the product was dried under reduced pressure. The residue was slurried in acetonitrile (15 mL) and filtered. The filtrate was concentrated under reduced pressure.

The crude product was purified by preparative LC-MS.

**Synthesis of Example 18:**

***Intermediate 18a):***

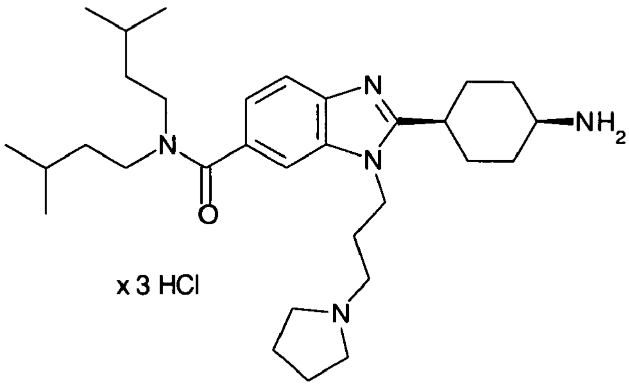

x 3 HCl

To a solution of intermediate 17a cis (100 mg, 0.164 mmol) in dioxane (2 mL) was added 4 N HCl in dioxane (6 mL) and methanol (1 mL). The reaction mixture was stirred at room temperature for 90 min. Volatiles were removed and the residue was co-evaporated twice with toluene (5 mL). The product was dried under reduced pressure.

***Example 18:***

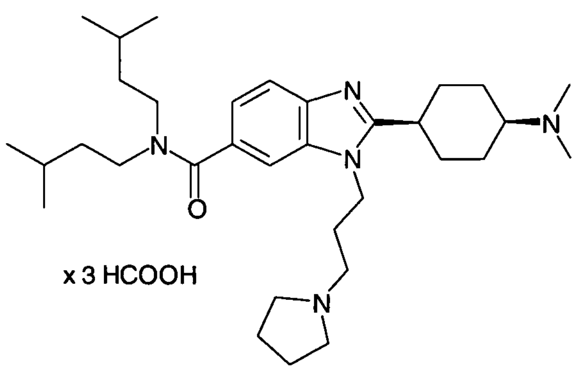

x 3 HCOOH

To a solution of intermediate 18a (91 mg, 0.147 mmol) in formic acid (2498 μL, 66.2 mmol) was added formaldehyde (36.5 % aq., 2816 μL, 36.8 mmol). The reaction mixture was stirred at 100°C overnight. Volatiles were removed and the product was dried under reduced pressure. The residue was slurried in acetonitrile (15 mL) and filtered. The filtrate was concentrated under reduced pressure.

The crude product was purified by preparative LC-MS.

**Synthesis of Example 19:**

***Intermediate 19a):***

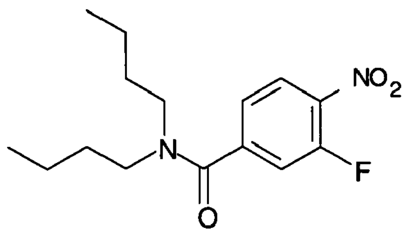

At 0°C to a solution of intermediate 3a (12.0 mmol) in dry DCM (100 mL) under argon atmosphere was added di-n-butylamine (2.14 mL, 12.6 mmol) and DIEA (2.19 mL, 12.6 mmol) dropwise and the reaction mixture was stirred at 0°C for 1 h and at room temperature overnight. The reaction mixture was poured into water (100 mL) and the organic layer was separated. The aqueous layer was extracted twice with DCM. The combined organics were washed three times with 1 N aqueous HCl solution and three times with saturated aqueous sodium bicarbonate solution, water and brine, dried over sodium sulfate and concentrated.

***Intermediate 19b):***

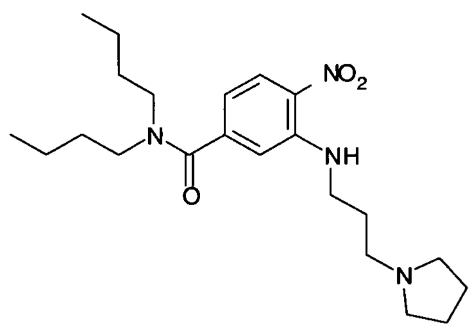

To N,N-dibutyl-3-fluoro-4-nitro-benzamide (3.40 g, 11.47 mmol) and potassium carbonate (3.17 g, 22.94 mmol) in acetonitrile (150 mL) was added 1-(3-aminopropyl)pyrrolidine (1596 μL, 12.62 mmol). The mixture was kept under reflux for 195 min and then concentrated at reduced pressure. The residue was taken up with DCM (200 mL) and water (100 mL). The aqueous phase was extracted twice with DCM and the combined organic fractions were washed with water and brine, dried over sodium sulfate and the solvent was removed under reduced pressure.

***Intermediate 19c):***

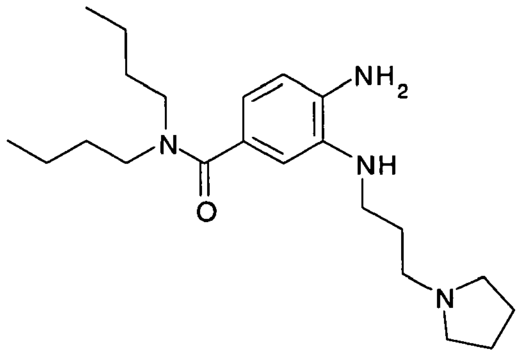

A mixture of intermediate 19b (11.47 mmol) and 10% palladium on charcoal (2.44 g, 2.29 mmol) in ethanol (70 mL) and ethyl acetate (70 mL) was evacuated and purged with argon three times and then evacuated and flushed with hydrogen three times. The reaction mixture was stirred at room temperature under atmospheric pressure of hydrogen for 3 h. The reaction mixture was filtered through Celite and the filter cake was washed with ethanol. The filtrate was evaporated.

The crude product was used for the next step without purification.

***Example 19:***

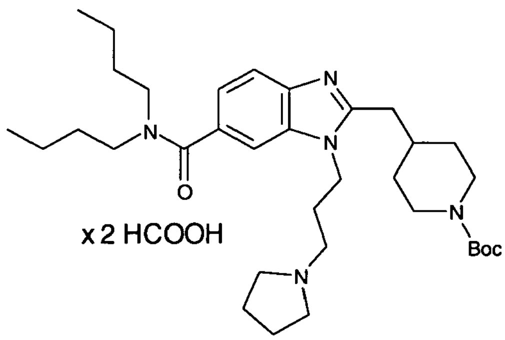

x 2 HCOOH

To a solution of intermediate 19c (869 mg, 2.32 mmol) in ethanol (50 mL) was added N-Boc-4-piperidineacetaldehyde (527 mg, 2.32 mmol). The reaction mixture was stirred vigorously at reflux temperature overnight. The solvent was removed under reduced pressure.

The crude product was purified column chromatography followed by preparative LC-MS.

**Synthesis of Example 20:**

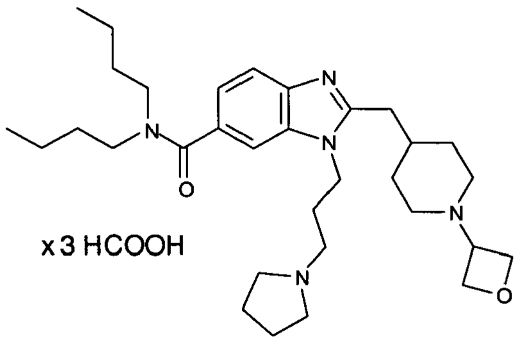

x 3 HCOOH

To a suspension of example 38 (170 mg, 0.288 mmol) in 1,2-dichloroethane (10 mL) was added triethylamine (120 μL, 0.864 mmol). The reaction mixture was stirred for 15 min, then 3-oxetanone (31 mg, 0.432 mmol) was added and stirring was continued for 15 min. Sodium triacetoxyborohydride (92 mg, 0.432 mmol) was added and the reaction mixture was stirred overnight. Additional 3-oxetanone (31 mg, 0.432 mmol) and sodium triacetoxyborohydride (92 mg, 0.432 mmol) were added and the reaction mixture was stirred overnight. Volatiles were removed and the residue was divided between with ethyl acetate (60 mL) and saturated aqueous sodium carbonate solution (20 mL). The organic layer was washed twice with saturated aqueous sodium carbonate solution (20 mL each). The aqueous layer was extracted back with ethyl acetate (20 mL). The combined organic layer was washed with brine, dried over sodium sulfate and the solvent was removed under reduced pressure.

The crude product was purified by preparative LC-MS.

**Synthesis of Example 21:**

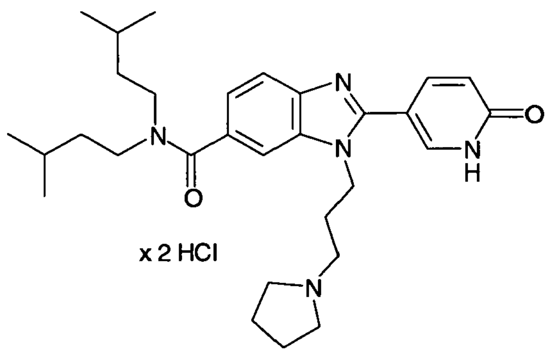

x 2 HCl

To a solution of intermediate 5b (403 mg, 1.00 mmol) in acetonitrile (20 mL) and acetic acid (200 μL) was added 6-hydroxynicotinaldehyde (123 mg, 1.00 mmol). The reaction mixture was stirred vigorously at reflux temperature overnight. Additional 6-hydroxynicotinaldehyde (123 mg, 1.00 mmol) was added and stirring at reflux temperature was continued overnight.The solvent was removed under reduced pressure.

The crude product was purified by preparative LC-MS.

The pure product (208 mg) was dissolved in methanol (5 mL) and 1 M HCl in diethyl ether (850 μL, 0.85 mmol) was added. The solvents were removed under reduced pressure. The product was taken up in water (10 mL) and lyophilized.

**Synthesis of Example 22:**

***Intermediate 22a):***

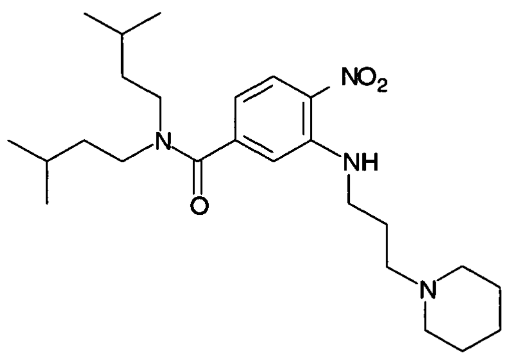

To 3-fluoro-N,N-bis-(3-methyl-butyl)-4-nitro-benzamide (500 mg, 1.54 mmol) and potassium carbonate (426 mg, 3.08 mmol) in acetonitrile (30 mL) was added piperidinopropylamine (236 mg, 1.85 mmol). The mixture was kept under reflux for 2.5 h and than concentrated at reduced pressure. The residue was taken up with DCM (40 mL) and water (20 mL). The aqueous phase was extracted twice with DCM and the combined organic fractions were washed with water and brine, dried over sodium sulfate and the solvent was removed under reduced pressure.

***Intermediate 22b):***

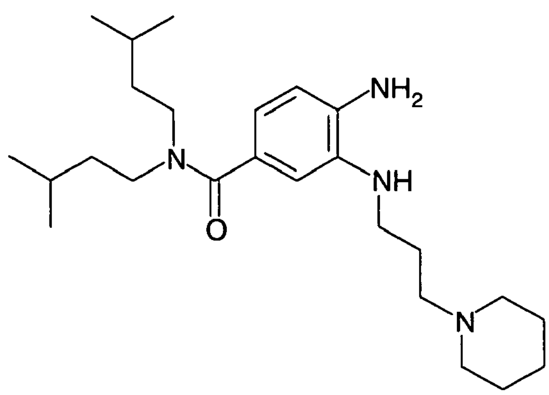

A mixture of intermediate 22a (635 mg, 1.42 mmol) and 10% palladium on charcoal (302 mg, 0.284 mmol) in ethanol (20 mL) and ethyl acetate (20 mL) was evacuated and purged with argon three times and then evacuated and flushed with hydrogen three times. The reaction mixture was stirred at room temperature under atmospheric pressure of hydrogen overnight. The reaction mixture was filtered through Celite and the filter cake was washed with ethanol. The filtrate was evaporated.

The crude product was used for the next step without purification.

***Example 22:***

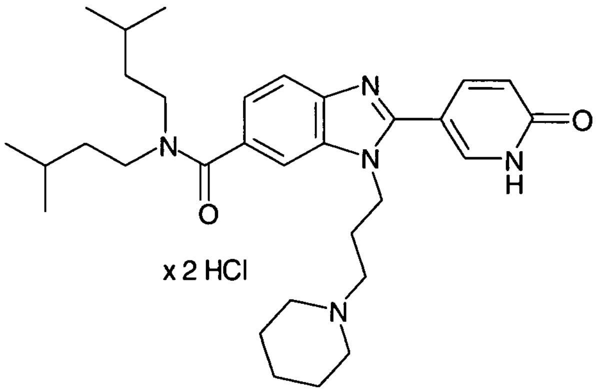

x 2 HCl

To a solution of intermediate 22b (295 mg, 0.720 mmol) in acetonitrile (20 mL) and acetic acid (200 μL) was added 6-hydroxynicotinaldehyde (87 mg, 0.708 mmol). The reaction mixture was stirred vigorously at reflux temperature overnight. The solvent was removed under reduced pressure.

The crude product was purified by preparative LC-MS.

The pure product (54 mg, orange oil) was dissolved in methanol (2 mL) and 1 M HCl in diethyl ether (110 μL, 0.110 mmol) was added. The solvents were removed under reduced pressure. The product was taken up in water (3 mL) and lyophilized.

**Synthesis of Example 23:**

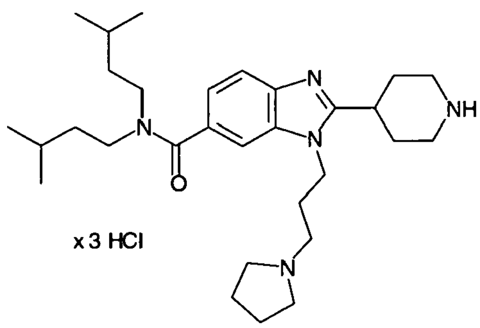

x 3 HCl

To a solution of example 11 (130 mg, 0.218 mmol) in dioxane (1 mL) was added 4 N HCl in dioxane (7 mL). The reaction mixture was stirred at room temperature for 30 min. Volatiles were removed and the residue was co-evaporated with toluene (7 mL). The residue was suspended in acetone (7 mL) and volatiles were removed. The product was dried under reduced pressure.

**Synthesis of Example 24:**

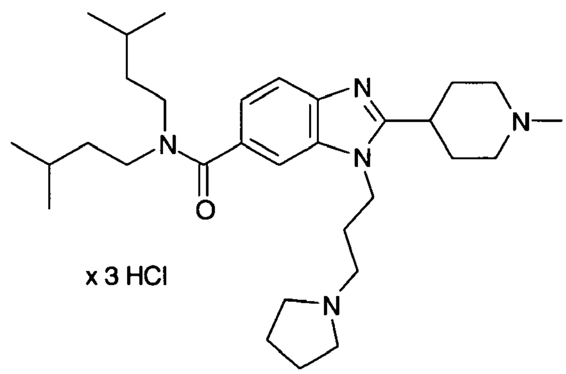

x 3 HCl

To a suspension of example 23 (125 mg, 0.207 mmol) in 1,2-dichloroethane (5 mL) was added triethylamine (86 μL, 0.621 mmol). The reaction mixture was stirred for 5 min, then formaldehyde (36.5% solution in water, 19 μL, 0.248 mmol) was added and stirring was continued for 5 min. Sodium triacetoxyborohydride (53 mg, 0.248 mmol) was added and the reaction mixture was stirred for 1 h. Volatiles were removed and the residue was divided between with ethyl acetate (60 mL) and saturated aqueous sodium carbonate solution (20 mL). The organic layer was washed twice with saturated aqueous sodium carbonate solution (20 mL each). The aqueous layer was extracted back with ethyl acetate (20 mL). The combined organic layer was washed with brine, dried over sodium sulfate and the solvent was removed under reduced pressure.

The pure product (98 mg, pale yellow oil) was dissolved in methanol (2 mL) and 1 M HCl in diethyl ether (385 μL, 0.385 mmol) was added. The solvents were removed under reduced pressure. The product was taken up in water (4 mL) and lyophilized.

**Synthesis of Example 25:**

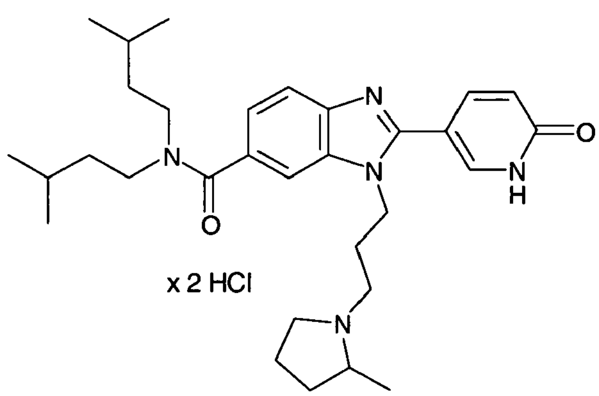

x 2 HCl

To a solution of intermediate 12e (417 mg, 1.00 mmol) in acetonitrile (20 mL) and acetic acid (200 μL) was added 6-hydroxynicotinaldehyde (123 mg, 1.00 mmol). The reaction mixture was stirred vigorously at reflux temperature overnight. The solvent was removed under reduced pressure.

The crude product was purified by preparative LC-MS.

The pure product (213 mg, 0.41 mmol, brown sticky solid) was dissolved in methanol (5 mL) and 1 M HCl in diethyl ether (820 μL, 0.82 mmol) was added. The solvents were removed under reduced pressure. The product was taken up in water (5 mL) and lyophilized.

**Synthesis of Example 26:**

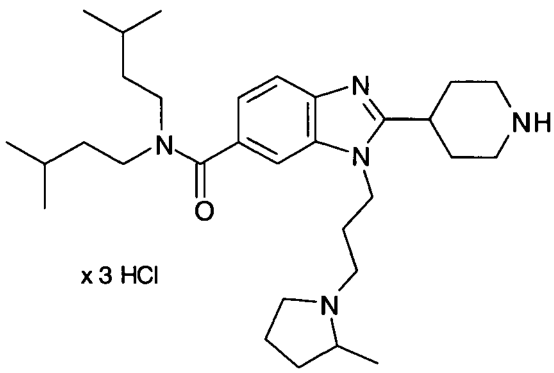

x 3 HCl

To a solution of example 12 (645 mg, 0.92 mmol) in dioxane (5 mL) was added 4 N HCl in dioxane (15 mL). The reaction mixture was stirred at room temperature for 30 min. Volatiles were removed and the residue was co-evaporated with toluene (15 mL). The residue was suspended in acetone (15 mL) and volatiles were removed. The product was dried under reduced pressure.

**Synthesis of Example 27:**

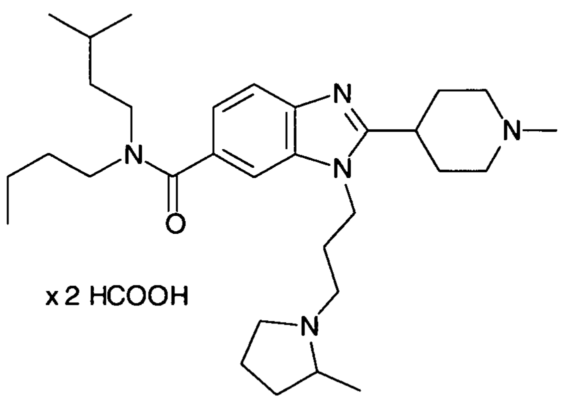

x 2 HCOOH

To a suspension of example 26 (136 mg, 0.220 mmol) in 1,2-dichloroethane (5 mlL was added triethylamine (92 μL, 0.660 mmol). The reaction mixture was stirred for 5 min, then formaldehyde (36.5% solution in water, 20 μL, 0.264 mmol) was added and stirring was continued for 5 min. Sodium triacetoxyborohydride (56 mg, 0.264 mmol) was added and the reaction mixture was stirred for 1 h. Volatiles were removed and the residue was divided between with ethyl acetate (60 mL) and saturated aqueous sodium carbonate solution (20 mL). The organic layer was washed twice with saturated aqueous sodium carbonate solution (20 mL each). The aqueous layer was extracted back with ethyl acetate (20 mL). The combined organic layer was washed with brine, dried over sodium sulfate and the solvent was removed under reduced pressure.

**Synthesis of Example 28:**

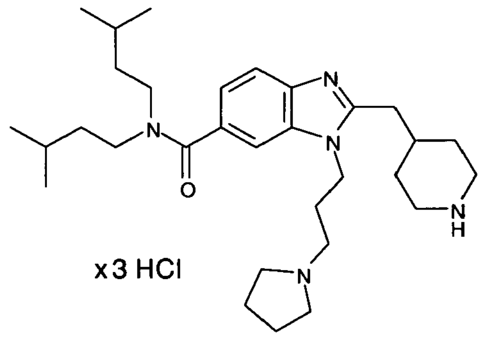

x 3 HCl

To a solution of example 13 (426 mg, 0.70 mmol) in dioxane (5 mL) was added 4 N HCl in dioxane (15 mL) and methanol (1 mL). The reaction mixture was stirred at room temperature for 45 min. Volatiles were removed and the residue was co-evaporated twice with toluene (10 mL). The product was dried under reduced pressure.

**Synthesis of Example 29:**

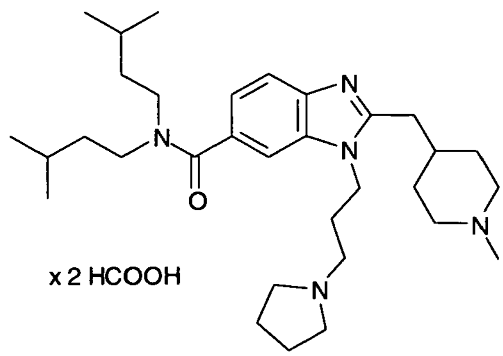

x 2 HCOOH

To a suspension of example 28 (136 mg, 0.220 mmol) in 1,2-dichloroethane (5 mL) was added triethylamine (92 µL, 0.660 mmol). The reaction mixture was stirred for 5 min, then formaldehyde (36.5% solution in water, 20 µL, 0.264 mmol) was added and stirring was continued for 5 min. Sodium triacetoxyborohydride (56 mg, 0.264 mmol) was added and the reaction mixture was stirred for 3.5 h. Volatiles were removed and the residue was divided between with ethyl acetate (60 mL) and saturated aqueous sodium carbonate solution (20 mL). The organic layer was washed twice with saturated aqueous sodium carbonate solution (20 mL each). The aqueous layer was extracted back with ethyl acetate (20 mL). The combined organic layer was washed with brine, dried over sodium sulfate and the solvent was removed under reduced pressure.

The crude product was purified by preparative LC-MS.

**Synthesis of Example 30:**

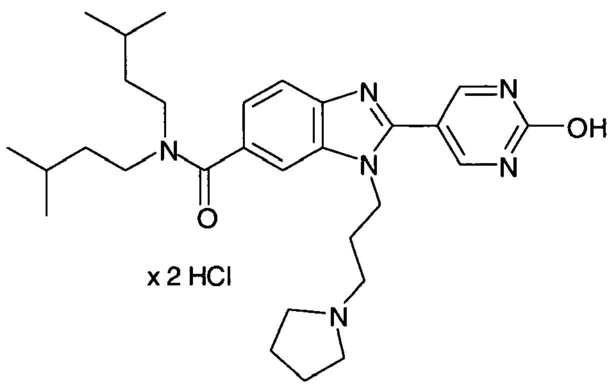

x 2 HCl

To a solution of intermediate 5b (403 mg, 1.00 mmol) in acetonitrile (20 mL) and acetic acid (200 μL) was added 2-hydroxypyrimidine-5-carbaldehyde (124 mg, 1.00 mmol). The reaction mixture was stirred vigorously at reflux temperature overnight. Additional 2-hydroxypyrimidine-5-carbaldehyde (124 mg, 1.00 mmol) was added and stirring at reflux temperature was continued overnight. The solvent was removed under reduced pressure. The residue was taken up with acetonitrile (50 mL) and filtered. The solvent was removed under reduced pressure.

The crude product was purified by preparative LC-MS.

**Synthesis of Example 31:**

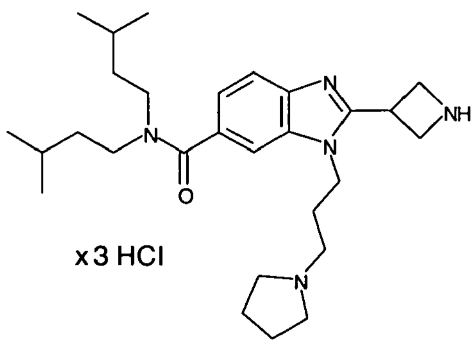

x 3 HCl

To a solution of example 16 (145 mg, 0.255 mmol) in dioxane (2 mL) was added 4 N HCl in dioxane (6 mL) and methanol (1 mL). The reaction mixture was stirred at room temperature for 2.5 h. Volatiles were removed and the residue was co-evaporated twice with toluene (5 mL). The product was dried under reduced pressure.

**Synthesis of Example 32:**

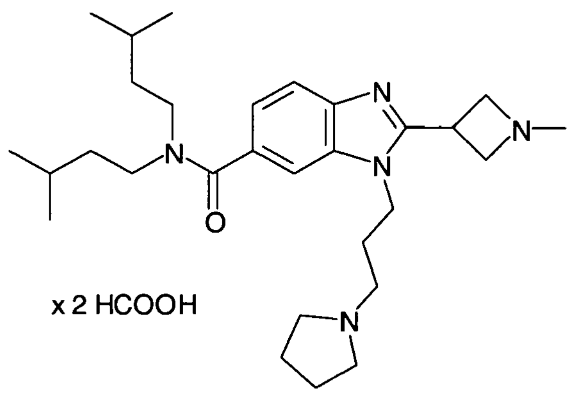

To a suspension of example 31 (143 mg, 0.248 mmol) in 1,2-dichloroethane (5 mL) was added triethylamine (104 μL, 0.744 mmol). The reaction mixture was stirred for 5 min, then formaldehyde (36.5% solution in water, 23 μL, 0.298 mmol) was added and stirring was continued for 5 min. Sodium triacetoxyborohydride (63 mg, 0.298 mmol) was added and the reaction mixture was stirred for 5.5 h. Additional formaldehyde (36.5% solution in water, 23 μL, 0.298 mmol) and sodium triacetoxyborohydride (63 mg, 0.298 mmol) were added and stirring was continued overnight. LC-MS still indicated the presence of some starting material. Volatiles were removed and the residue was divided between ethyl acetate (60 mL) and saturated aqueous sodium carbonate solution (20 mL). The organic layer was washed twice with saturated aqueous sodium carbonate solution (20 mL each). The aqueous layer was extracted back with ethyl acetate (20 mL). The combined organic layer was washed with brine, dried over sodium sulfate and the solvent was removed under reduced pressure.

The crude product was purified by preparative LC-MS.

**Synthesis of Example 33:**

***Intermediate 33a):***

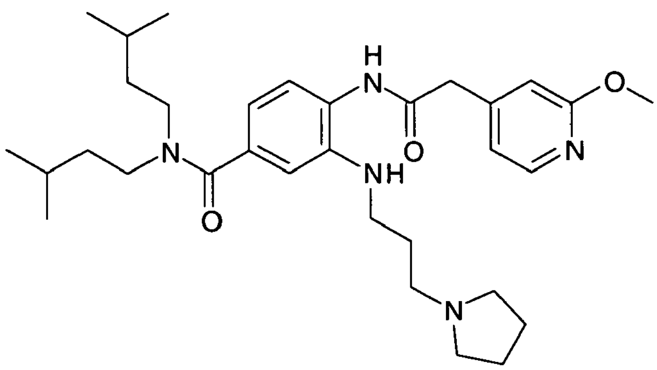

To a solution of intermediate 5b (201 mg, 0.500 mmol) in DCM (10 mL) was added (2-methoxypyridin-4-yl) acetic acid (88 mg, 0.525 mmol), EDC (101 mg, 0.525 mmol) and DMAP (5 mg) and the solution was stirred at room temperature for 20 h. The reaction mixture was diluted with DCM (50 mL) and extracted twice with water, saturated sodium bicarbonate solution and with brine. The organic layer was dried over sodium sulfate and the solvent was removed under reduced pressure.

The crude product was purified by column chromatography.

***Example 33:***

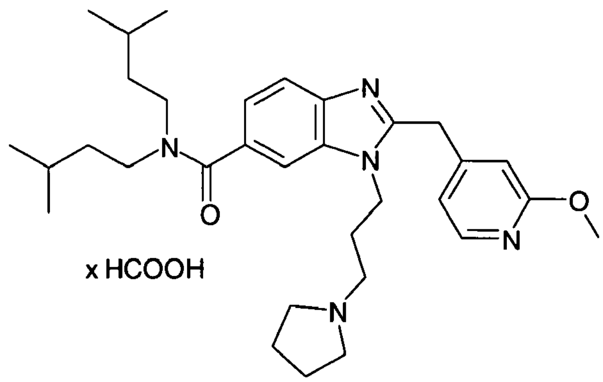

x HCOOH

Intermediate 33a (218 mg, 0.395 mmol) was dissolved in acetic acid (5 mL) and stirred at 80˚C for 4 h. The solvent was removed under reduced pressure.

The crude product was purified by preparative LC-MS.

**Synthesis of Example 34:**

***Intermediate 34a):***

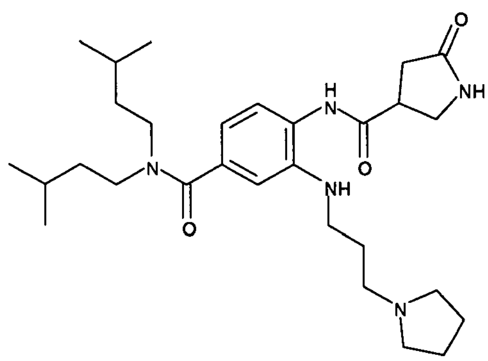

To a solution of intermediate 5b (200 mg, 0.496 mmol) in DCM (10 mL) was added 5-oxopyrrolidine-3-carboxylic acid (68 mg, 0.526 mmol), EDC (101 mg, 0.527 mmol) and DMAP (5 mg) and the solution was stirred at room temperature for 20 h. A second addition of 5-oxopyrrolidine-3-carboxylic acid (32 mg) was performed and it was stirred over night. A third addition of 5-oxopyrrolidine-3-carboxylic acid (68 mg) and EDC (101 mg) was performed and it was stirred over night. The reaction mixture was diluted with DCM (50 mL) and extracted twice with water, saturated sodium bicarbonate solution and with brine. The organic layer was dried over sodium sulfate and the solvent was removed under reduced pressure.

The crude product was purified by column chromatography.

***Example 34:***

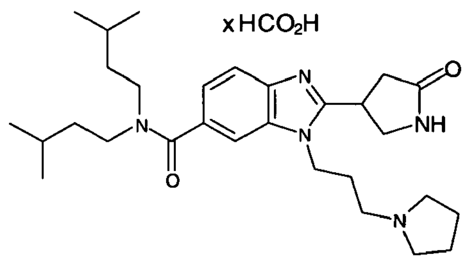

Intermediate 34a (184 mg, 0.358 mmol) was dissolved in acetic acid (5 mL) and stirred at 80˚C over night. The solvent was removed under reduced pressure.

The crude product was purified by preparative LC-MS.

**Synthesis of Example 35:**

***Intermediate 35a):***

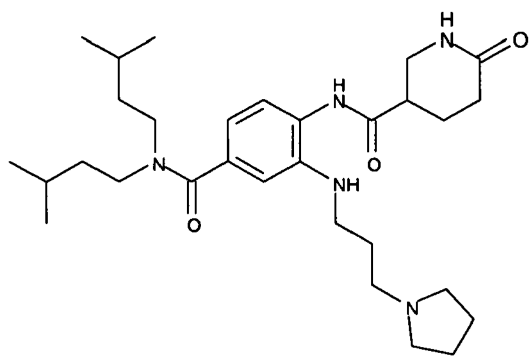

To a solution of intermediate 5b (100 mg, 0.248 mmol) in DCM (5 mL) was added 6-oxopiperidine-3-carboxylic acid (38 mg, 0.265 mmol), EDC (51 mg, 0.266 mmol) and DMAP (2.5 mg) and the solution was stirred at room temperature for 5 h. A second addition of 6-oxopiperidine-3-carboxylic acid (38 mg), EDC (51 mg), and DMAP (2.5 mg) was performed and it was stirred over night. A third addition of 6-oxopiperidine-3-carboxylic acid (38 mg) and EDC (51 mg) was performed and it was stirred over night. The reaction mixture was diluted with DCM (50 mL) and extracted twice with water, saturated sodium bicarbonate solution and with brine. The organic layer was dried over sodium sulfate and the solvent was removed under reduced pressure.

The crude product was purified by column chromatography.

***Example 35:***

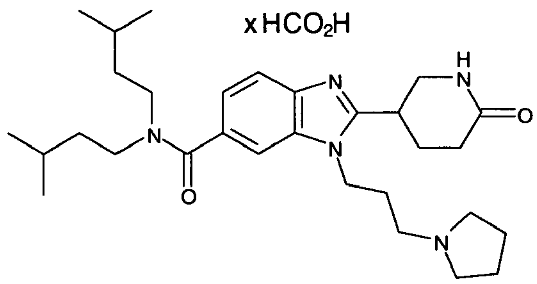

Intermediate 35a (116 mg, 0.2198 mmol) was dissolved in acetic acid (3 mL) and stirred at 80°C over night. The solvent was removed under reduced pressure.

The crude product was purified by preparative LC-MS.

**Synthesis of Example 36:**

***Intermediate 36a):***

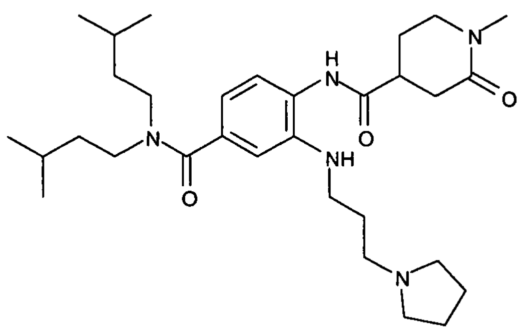

To a solution of intermediate 5b (100 mg, 0.248 mmol) in DCM (5 mL) was added 1-methyl-2-oxopiperidine-4-carboxylic acid (41 mg, 0.261 mmol), EDC (51 mg, 0.266 mmol) and DMAP (2.5 mg) and the solution was stirred at room temperature for 16 h. A second addition of 1-methyl-2-oxopiperidine-4-carboxylic acid (41 mg), EDC (51 mg) was performed and it was stirred over night. The reaction mixture was diluted with DCM (50 mL) and extracted twice with water, saturated sodium bicarbonate solution and with brine. The organic layer was dried over sodium sulfate and the solvent was removed under reduced pressure.

The crude product was purified by column chromatography.

***Example 36:***

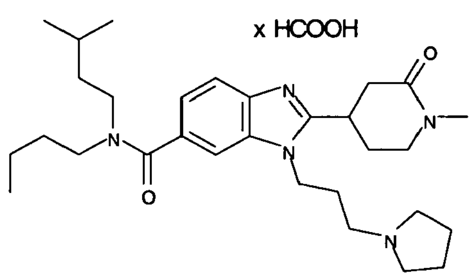

Intermediate 36a (121 mg, 0.223 mmol) was dissolved in acetic acid (3 mL) and stirred at 80°C over night. The solvent was removed under reduced pressure.
The crude product was purified by preparative LC-MS.

**Synthesis of Example 37:**

***Intermediate 37a):***

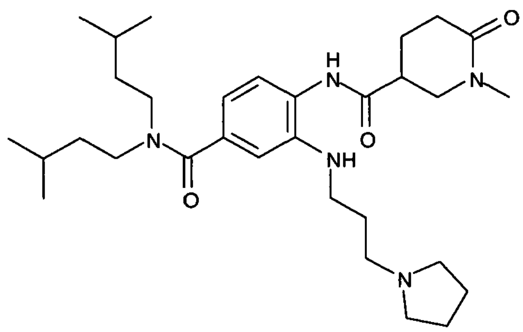

To a solution of intermediate 5b (100 mg, 0.248 mmol) in DCM (5 mL) was added 1-methyl-6-oxopiperidine-3-carboxylic acid (41 mg, 0.261 mmol), EDC (51 mg, 0.266 mmol) and DMAP (2.5 mg) and the solution was stirred at room temperature for 16 h. A second addition of 1-methyl-6-oxopiperidine-3-carboxylic acid (41 mg), EDC (51 mg), and DMAP (2.5 mg) was performed and it was stirred over the week end. A third addition of 1-methyl-6-oxopiperidine-3-carboxylic acid (38 mg) and EDC (51 mg) was performed and it was stirred over night. The reaction mixture was diluted with DCM (50 mL) and extracted twice with water, saturated sodium bicarbonate solution and with brine. The organic layer was dried over sodium sulfate and the solvent was removed under reduced pressure.
The crude product was purified by column chromatography.

***Example 37:***

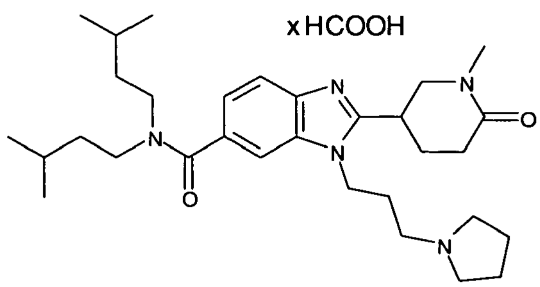

Intermediate 37a (104 mg, 0.192 mmol) was dissolved in acetic acid (3 mL) and stirred at 80°C over night. The solvent was removed under reduced pressure.
The crude product was purified by preparative LC-MS.

**Synthesis of Example 38:**

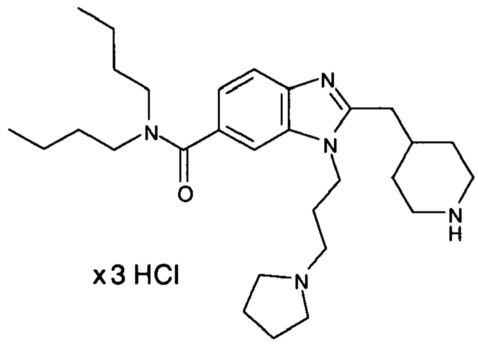

x 3 HCl

To a solution of example 19 (525 mg, 0.78 mmol) in dioxane (5 mL) was added 4 N HCl in dioxane (15 mL) and methanol (1 mL). The reaction mixture was stirred at room temperature for 1 h. Volatiles were removed and the residue was co-evaporated twice with toluene (10 mL). The product was dried under reduced pressure.

**Synthesis of Example 39:**

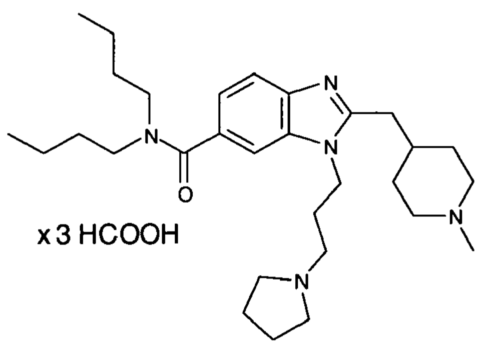

x 3 HCOOH

To a suspension of example 38 (170 mg, 0.288 mmol) in 1,2-dichloroethane (10 mL) was added triethylamine (120 μL, 0.864 mmol). The reaction mixture was stirred for 15 min, then formaldehyde solution, 36.5% in water (33 μL, 0.432 mmol) was added and stirring was continued for 15 min. Sodium triacetoxyborohydride (92 mg, 0.432 mmol) was added and the reaction mixture was stirred overnight. Additional formaldehyde solution, 36.5% in water (33 μL, 0.432 mmol) and sodium triacetoxyborohydride (92 mg, 0.432 mmol) were added and the reaction mixture was stirred overnight. Volatiles were removed and the residue was divided between ethyl acetate (60 mL) and saturated aqueous sodium carbonate solution (20 mL). The organic layer was washed twice with saturated aqueous sodium carbonate solution (20 mL each). The aqueous layer was extracted back with ethyl acetate (20 mL). The combined organic layer was washed with brine, dried over sodium sulfate and the solvent was removed under reduced pressure.

The crude product was purified by preparative LC-MS.

**BIOLOGICAL ASSAYS**

**A. Binding Assay**

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.

The degree of fluorescence polarization is determined with a fluorescence polarization microplate reader.

**B. Functional Assay**

Agonistic activity of human melanocortin receptors is determined in a homogeneous membrane based assay. Competition between unlabeled cAMP and a fixed quantity of fluorescence labeled cAMP for a limited number of binding sites on a cAMP specific antibody is revealed by fluorescence polarization.

The test compound or unlabeled NDP-alpha-MSH is dispensed at varying concentrations to a 384 well microtiter plate. Membrane preparations from HEK293 cells expressing the human melanocortin receptors are added. After a short preincubation period, an appropriate amount of ATP, GTP and the cAMP antibody is added and the plate is further incubated before the fluorescence labeled cAMP conjugate is dispensed. The plate is incubated for 2 h at 4˚C before it is read on a fluorescence polarization microplate reader. The amount of cAMP produced as a response to a test compound is compared to the production of cAMP resulting from stimulation with NDP-alpha-MSH.

Representative compounds of the present invention were tested and found to bind to the melanocortin-4 receptor. These compounds were generally found to have $IC_{50}$ values less than 2 μM

**Table 8: Biological data for selected examples of the invention**

In the table are listed the $IC_{50}$ values of the hMC-4R binding assay and the $EC_{50}$ values of the functional assay. The $IC_{50}$ and $EC_{50}$ values were grouped in 3 classes:
a ≤ 0.1 μM; b > 0. μM and ≤ 1.0 μM; c > 1.0 μM and ≤10 μM

| Example | hMC-4R binding assay $IC_{50}$/μM | hMC-4R functional assay $EC_{50}$/μM | % activation functional assay |
|---|---|---|---|
| SHU9119 | a | - | 7 |
| NDP-α-MSH | a | a | 100 |
| 1 | b | - | -15 |
| 2 | a | - | -11 |
| 3 | a | b | -25 |
| 4 | a | a | -28 |
| 5 | b | - | -6 |
| 6 | b | - | -11 |
| 7 | c | - | -4 |
| 8 | a | - | -6 |
| 9 | a | - | -9 |
| 10 | b | - | 0 |
| 11 | a | a | -33 |
| 12 | a | a | -40 |
| 13 | a | - | -7 |
| 14 | a | - | -18 |
| 15 | a | - | -11 |
| 16 | a | - | -9 |
| 17 | a | - | -6 |
| 18 | a | - | -19 |
| 19 | a | a | -33 |
| 20 | b | - | 0 |
| 21 | a | - | -14 |
| 22 | a | - | -12 |

(continued)

| Example | hMC-4R binding assay $IC_{50}/\mu M$ | hMC-4R functional assay $EC_{50}/\mu M$ | % activation functional assay |
|---|---|---|---|
| 23 | a | a | -39 |
| 24 | a | a | -44 |
| 25 | a | a | -37 |
| 26 | a | - | -12 |
| 27 | a | - | -2 |
| 28 | a | - | -6 |
| 29 | a | - | -10 |
| 30 | b | - | -12 |
| 31 | a | b | -32 |
| 32 | a | a | -18 |
| 33 | a | - | 0 |
| 34 | a | - | -10 |
| 35 | b | - | -11 |
| 36 | b | - | -5 |
| 37 | b | - | -2 |
| 38 | a | - | -5 |
| 39 | a | - | -8 |

## C. In Vivo Food Intake Models

### 1. Spontaneous Feeding Paradigm

Food intake in rats is measured after s.c., i.p. or p.o. administration of the test compound (see e.g. Chen, A.S. et al. Transgenic Res 2000 Apr;9(2):145-54).

### 2. Models of LPS-Induced Anorexia and Tumor-Induced Cachexia

Prevention or amelioration of anorexia induced by lipopolysaccharide (LPS) administration or cachexia induced by tumor growth is determined upon s.c., i.p. or p.o. administration of test compounds to rats (see e.g. Marks, D.L.; Ling, N and Cone, R.D. Cancer Res 2001 Feb 15;61 (4):1432-8).

## D. In Vivo Model for Depression

### Forced Swim Test in Mice

### Principle

Animals placed in a container filled with water show periods of increased swimming activity and periods of relative immobility. Clinically active anti-depressants have been found to delay the onset of the first phase of immobility and to reduce the total time of relative immobility. The list of active compounds includes monoamino-oxidase-A (MAO-A) inhibitors such as moclobemide, brofaromine, noradrenaline (NA) uptake inhibitors such as imipramine and amytryptilin, MAO-B inhibitors such as selegiline and tranylcypromine, serotonin uptake inhibitors (SSRI) such as fluoxetine and paroxetine and combined NA / SSRI such as venlafaxine. Benzodiazepines and other types of psychoactive compounds have been found to be inactive in this test (see e.g. Porsolt R.D., Bertin A., Jaffre M. Behavioral despair in rats and mice: strain differences and the effect of imipramine. Eur. J. Pharmacol. 1978, 51: 291-294, Borsini F. and Meli A. Is the forced swimming test a suitable model for revealing antidepressant activity (Psychopharmacol. 1988, 94: 147-160).

**Experimental Procedure**

Subjects to be used are male Swiss mice (4-5 weeks old). Animals are randomly assigned to different groups (10 mice per group).

Each animal is placed individually in the water bath where it remains for 6 minutes. The animal is given an accommodation period of 2 minutes. During the subsequent 4 minutes observation period, the duration of the periods of immobility is recorded. In addition, the frequency of the immobility state is also measured. The mouse is considered to be immobile when it passively floats on the water making only small movements to keep its head above the surface.

The water is replaced with clean water after 3 animals tested.

**Drug Administration**

Treatment is administered before the test as vehicle or test compound at different doses. Compounds are usually administered by p.o. i.p. or s.c. routes.

**Data Analysis**

Analysis of data is performed using ANOVA followed by Fisher's PLSD test as post-hoc test.

**E. In vitro ADME Assays**

**1. Microsomal Stability**

**Experimental Procedure**

Pooled human liver microsomes (pooled male and female) and pooled rat liver microsomes (male Sprague Dawley rats) are prepared. Microsomes are stored at -80˚C prior to use.

Microsomes (final concentration 0.5 mg/ml), 0.1 M phosphate buffer pH7.4 and test compound (final substrate concentration = 3 μM; final DMSO concentration = 0.25%) are pre-incubated at 37˚C prior to the addition of NADPH (final concentration = 1 mM) to initiate the reaction. The final incubation volume is 25 μl. A control incubation is included for each compound tested where 0.1 M phosphate buffer pH7.4 is added instead of NADPH (minus NADPH). Two control compounds are included with each species. All incubations are performed singularly for each test compound.

Each compound is incubated for 0, 5, 15, 30 and 45 min. The control (minus NADPH) is incubated for 45 min only. The reactions are stopped by the addition of 50 μl methanol containing internal standard at the appropriate time points. The incubation plates are centrifuged at 2,500 rpm for 20 min at 4˚C to precipitate the protein.

**Quantitative Analysis**

Following protein precipitation, the sample supernatants are combined in cassettes of up to 4 compounds and analysed using generic LC-MS/MS conditions.

**Data Analysis**

From a plot of the peak area ratio (compound peak area/internal standard peak area) against time, the gradient of the line is determined. Subsequently, half-life and intrinsic clearance are calculated using the equations below:

Elimination rate constant (k) = (- gradient)

Half life ($t_{1/2}$) (min) =

$$\frac{0.693}{k}$$

Intrinsic Clearance ($CL_{int}$) (μl/min/mg protein) =

$$\frac{V \times 0.693}{t_{1/2}}$$

where V = Incubation volume µl/mg microsomal protein.

Two control compounds are included in the assay and if the values for these compounds are not within the specified limits the results are rejected and the experiment repeated.

**2. Hepatocyte Stability**

**Experimental Procedure**

Suspensions of cryopreserved hepatocytes are used for human hepatocyte stability assay (pooled from 3 individuals). All cryopreserved hepatocytes are purchased from In Vitro Technologies, Xenotech or TCS.
Incubations are performed at a test or control compound concentration of 3 µM at a cell density of $0.5 \times 10^6$ viable cells/mlL. The final DMSO concentration in the incubation is 0.25%. Control incubations are also performed in the absence of cells to reveal any non-enzymatic degradation.
Duplicate samples (50 µl) are removed from the incubation mixture at 0, 5, 10, 20, 40 and 60 min (control sample at 60 min only) and added to methanol, containing internal standard (100 µl), to stop the reaction.
Tolbutamide, 7-hydroxycoumarin, and testosterone are used as control compounds.
The samples are centrifuged (2500 rpm at 4˚C for 20 min) and the supernatants at each time point are pooled for cassette analysis by LC-MS/MS using generic methods.

**Data Analysis**

From a plot of In peak area ratio (compound peak area/internal standard peak area) against time, the gradient of the line is determined. Subsequently, half-life and intrinsic clearance are calculated using the equations below:

Elimination rate constant (k) = (- gradient)

Half life ($t_{1/2}$)(min) =

$$\frac{0.693}{k}$$

Intrinsic Clearance ($CL_{int}$)(µl/min/million cells) =

$$\frac{V \times 0.693}{t_{1/2}}$$

where V = Incubation volume (µl)/number of cells

**3. Caco-2 Permeability (Bi-directional)**

**Experimental Procedure**

Caco-2 cells obtained from the ATCC at passage number 27 are used. Cells (passage number 40-60) are seeded on to Millipore Multiscreen Caco-2 plates at 1 x 105 cells/$cm^2$. They are cultured for 20 days in DMEM and media is changed every two or three days. On day 20 the permeability study is performed.
Hanks Balanced Salt Solution (HBSS) pH7.4 buffer with 25 mM HEPES and 10 mM glucose at 37˚C is used as the medium in permeability studies. Incubations are carried out in an atmosphere of 5% $CO_2$ with a relative humidity

of 95%.

On day 20, the monolayers are prepared by rinsing both basolateral and apical surfaces twice with HBSS at 37°C. Cells are then incubated with HBSS in both apical and basolateral compartments for 40 min to stabilize physiological parameters.

HBSS is then removed from the apical compartment and replaced with test compound dosing solutions. The solutions are made by diluting 10 mM test compound in DMSO with HBSS to give a final test compound concentration of 10 μM (final DMSO concentration 1%). The fluorescent integrity marker lucifer yellow is also included in the dosing solution. Analytical standards are made from dosing solutions. Test compound permeability is assessed in duplicate. On each plate compounds of known permeability characteristics are run as controls. The apical compartment inserts are then placed into 'companion' plates containing fresh HBSS. For basolateral to apical (B-A) permeability determination the experiment is initiated by replacing buffer in the inserts then placing them in companion plates containing dosing solutions. At 120 min the companion plate is removed and apical and basolateral samples diluted for analysis by LC-MS/MS. The starting concentration ($C_0$) and experimental recovery is calculated from both apical and basolateral compartment concentrations.

The integrity of the monolayers throughout the experiment is checked by monitoring lucifer yellow permeation using fluorimetric analysis. Lucifer yellow permeation is low if monolayers have not been damaged. Test and control compounds are quantified by LC-MS/MS cassette analysis using a 5-point calibration with appropriate dilution of the samples. Generic analytical conditions are used.

If a lucifer yellow $P_{app}$ value is above QC limits in one individual test compound well, then an n=1 result is reported. If lucifer yellow $P_{app}$ values are above QC limits in both replicate wells for a test compound, the compound is re-tested. Consistently high lucifer yellow permeation for a particular compound in both wells indicates toxicity. No further experiments are performed in this case.

**Data Analysis**

The permeability coefficient for each compound ($P_{app}$) is calculated from the following equation:

$$P_{app} = \frac{dQ/dt}{C_0 \times A}$$

Where dQ/dt is the rate of permeation of the drug across the cells, $C_0$ is the donor compartment concentration at time zero and A is the area of the cell monolayer. $C_0$ is obtained from analysis of donor and receiver compartments at the end of the incubation period. It is assumed that all of the test compound measured after 120 min incubation was initially present in the donor compartment at 0 min. An asymmetry index (AI) is derived as follows:

$$AI = \frac{P_{app}\,(B\text{-}A)}{P_{app}\,(A\text{-}B)}$$

An asymmetry index above unity shows efflux from the Caco-2 cells, which indicates that the compound may have potential absorption problems in vivo.

The apparent permeability ($P_{app}$ (A-B)) values of test compounds are compared to those of control compounds, atenolol and propranolol, that have human absorption of approximately 50 and 90% respectively (Zhao, Y.H., et al., (2001). Evaluation of Human Intestinal Absorption Data and Subsequent Derivation of a Quantitative Structure-Activity Relationship (QSAR) with the Abraham Descriptors. Journal of Pharmaceutical Sciences. 90 (6), 749-784). Talinolol (a known P-gp substrate (Deferme, S., Mols, R., Van Driessche, W., Augustijns, P. (2002). Apricot Extract Inhibits the P-gp-Mediated Efflux of Talinolol. Journal of Pharmaceutical Sciences. 91 (12), 2539-48)) is also included as a control compound to assess whether functional P-gp is present in the Caco-2 cell monolayer.

### 4. Cytochrome P450 Inhibition (5 Isoform $IC_{50}$ Determination))

#### Experimental Procedure

#### CYP1 A Inhibition

Six test compound concentrations (0.05, 0.25, 0.5, 2.5, 5, 25 μM in DMSO; final DMSO concentration = 0.35%) are incubated with human liver microsomes (0.25 mg/ml) and NADPH (1 mM) in the presence of the probe substrate ethoxyresorufin (0.5 μM) for 5 min at 37°C. The selective CYP1 A inhibitor, alpha-naphthoflavone, is screened alongside the test compounds as a positive control.

#### CYP2C9 Inhibition

Six test compound concentrations (0.05, 0.25, 0.5, 2.5, 5, 25 μM in DMSO; final DMSO concentration = 0.25%) are incubated with human liver microsomes (1 mg/ml) and NADPH (1 mM) in the presence of the probe substrate tolbutamide (120 μM) for 60 min at 37°C. The selective CYP2C9 inhibitor, sulphaphenazole, is screened alongside the test compounds as a positive control.

#### CYP2C19 Inhibition

Six test compound concentrations (0.05, 0.25, 0.5, 2.5, 5, 25 μM in DMSO; final DMSO concentration = 0.25%) are incubated with human liver microsomes (0.5 mg/ml) and NADPH (1 mM) in the presence of the probe substrate mephenytoin (25 μM) for 60 min at 37°C. The selective CYP2C19 inhibitor, tranylcypromine, is screened alongside the test compounds as a positive control.

#### CYP2D6 Inhibition

Six test compound concentrations (0.05, 0.25, 0.5, 2.5, 5, 25 μM in DMSO; final DMSO concentration = 0.25%) are incubated with human liver microsomes (0.5 mg/ml) and NADPH (1 mM) in the presence of the probe substrate dextromethorphane (5 μM) for 30 min at 37°C. The selective CYP2D6 inhibitor, quinidine, is screened alongside the test compounds as a positive control.

#### CYP3A4 Inhibition

Six test compound concentrations (0.05, 0.25, 0.5, 2.5, 5, 25 μM in DMSO; final DMSO concentration 0.26%) are incubated with human liver microsomes (0.25 mg/ml) and NADPH (1 mM) in the presence of the probe substrate midazolam (2.5 μM) for 5 min at 37°C. The selective CYP3A4 inhibitor, ketoconazole, is screened alongside the test compounds as a positive control.

For the CYP1 A incubations, the reactions are terminated by the addition of methanol, and the formation of the metabolite, resorufin, is monitored by fluorescence (excitation wavelength = 535 nm, emission wavelength = 595 nm). For the CYP2C9, CYP2C19, CYP2D6, and CYP3A4 incubations, the reactions are terminated by the addition of methanol containing internal standard. The samples are then centrifuged, and the supernatants are combined, for the simultaneous analysis of 4-hydroxytolbutamide, 4-hydroxymephenytoin, dextrorphan, and 1-hydroxymidazolam plus internal standard by LC-MS/MS. Generic LC-MS/MS conditions are used. Formic acid in deionised water (final concentration = 0.1 %) is added to the final sample prior to analysis. A decrease in the formation of the metabolites compared to vehicle control is used to calculate an $IC_{50}$ value (test compound concentration which produces 50% inhibition).

### 5. Plasma Protein Binding (10%)

#### Experimental Procedure

Solutions of test compound (5 μM, 0.5% final DMSO concentration) are prepared in buffer (pH 7.4) and 10% plasma (v/v in buffer). The experiment is performed using equilibrium dialysis with the two compartments separated by a semi-permeable membrane. The buffer solution is added to one side of the membrane and the plasma solution to the other side. Standards are prepared in plasma and buffer and are incubated at 37°C. Corresponding solutions for each compound are analyzed in cassettes by LC-MS/MS.

**Quantitative Analysis**

After equilibration, samples are taken from both sides of the membrane. The solutions for each batch of compounds are combined into two groups (plasma-free and plasma-containing) then cassette analyzed by LC-MS/MS using two sets of calibration standards for plasma-free (7 points) and plasma-containing solutions (6 points). Generic LC-MS/MS conditions are used. Samples are quantified using standard curves prepared in the equivalent matrix. The compounds are tested in duplicate.

A control compound is included in each experiment.

**Data Analysis**

$$fu = \frac{1 - ((PC - PF))}{(PC)}$$

fu = fraction unbound

PC = sample concentration in protein containing side

PF = sample concentration in protein free side

fu at 10% plasma is converted to fu 100% plasma using the following equation:

$$fu_{100\%} = \frac{fu_{10\%}}{10-(9*fu_{10\%})}$$

**Examples of a Pharmaceutical Composition**

As a specific embodiment of an oral composition of a compound of the present invention, 35 mg of Example 32 is formulated with sufficient finely divided lactose to provide a total amount of 580 to 590 mg to fill a size 0 hard gelatin capsule.

As another specific embodiment of an oral composition of a compound of the present invention, 33 mg of Example 4 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the spirit and scope of the invention. For example, effective dosages, other than the preferred doses as set forth above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

**Claims**

**1.** A compound according to formula (I)

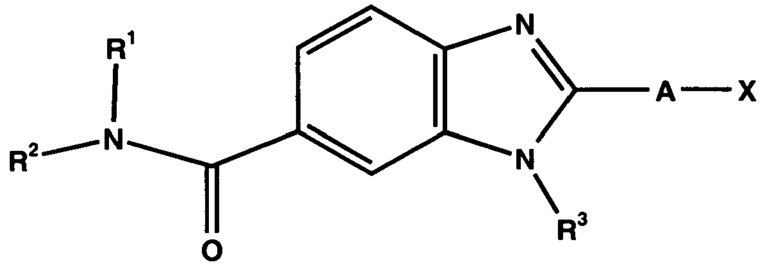

(I)

and enantiomers, diastereomers, tautomers, solvates and pharmaceutically acceptable salts thereof, wherein
$R^1$ and $R^2$ are independently from each other selected from

H,
$C_{1-6}$alkyl,
$C_{1-6}$alkylene-O-$C_{1-6}$alkyl,
$C_{1-3}$alkylene-heterocyclyl, and
$C_{1-6}$alkylene-$C_{3-7}$cycloalkyl, or

$R^1$ and $R^2$, together with the nitrogen atom to which they are attached to, form a 5 to 6- membered ring which may additionally contain one oxygen atom in the ring and which is unsubstituted or substituted by one or more substituents selected from OH, $C_{1-6}$alkyl, O-$C_{1-6}$alkyl, $C_{0-3}$alkylene-$C_{3-5}$cycloalkyl, $C_{1-6}$alkylene-O-$C_{1-6}$alkyl and $(CH_2)_{0-3}$-phenyl;
A is -$C_{1-6}$alkylene,

-$C_{2-6}$alkenylene,
-$C_{2-6}$alkinylene or
a bond
wherein alkylene, alkenylene and alkinylene are unsubstituted or substituted with one ore more $R^4$ or two alkyl chains attached to the same carbon atom of the alkylene, alkenylene or alkinylene form a 3-6 membered ring;

$R^4$ is independently selected from

$C_{1-6}$alkyl,
O-$C_{1-6}$alkyl,
OH,
halogen, and
$C_{3-6}$cycloalkyl;

X is 4 to 6-membered saturated heterocyclyl selected from the group consisting of $X^1$ to $X^7$

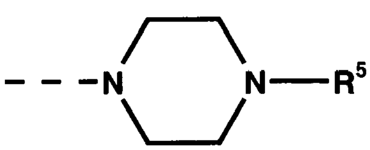

$X^1$,

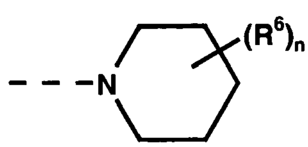

$X^2$,

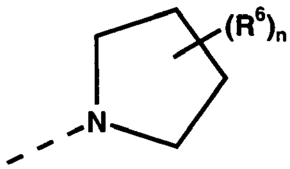
X$^3$,

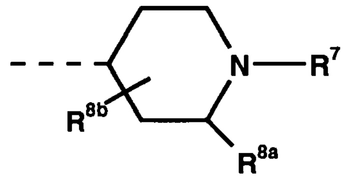
X$^4$,

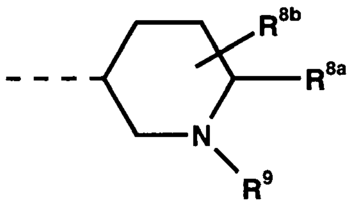
X$^5$,

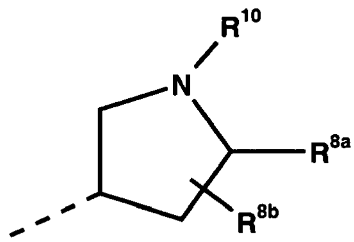
X$^6$ and

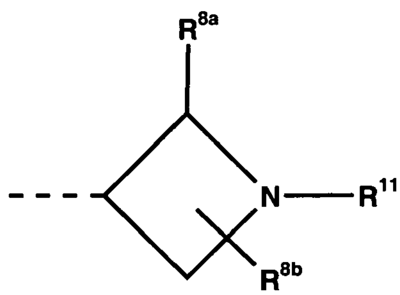
X$^7$,

5- to 6-membered heteroaryl selected from the group consisting of X$^8$ to X$^{13}$

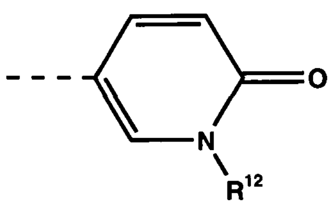
X$^8$,

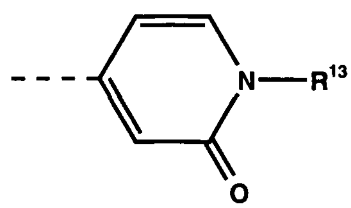
X$^9$,

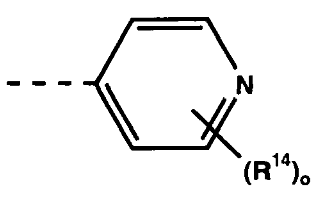
X$^{10}$,

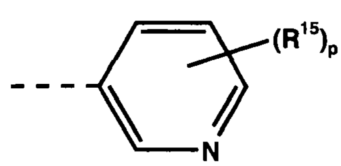
X$^{11}$,

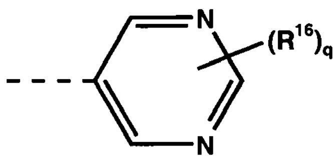 $X^{12}$, and 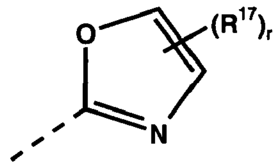 $X^{13}$,

$NH(C_{1-6}alkyl)$,
$N(C_{1-6}alkyl)_2$ or
$C_{3-8}$cycloalkyl, substituted with one or more $R^{18}$;

$R^5$ is H,

$C_{1-6}$ alkyl, unsubstituted or substituted with one or more substituents independently selected from OH, halogen and O-$C_{1-6}$alkyl,
C(O)-$C_{1-6}$alkyl,
$COOC_{1-6}$alkyl, or
4 to 6 membered saturated heterocycle having in the ring 1 or 2 heteroatoms independently selected from O, N and S;

$R^6$ is $C_{1-6}$ alkyl, unsubstituted or substituted with one or more substituents independently selected from OH, halogen, O-$C_{1-6}$alkyl and C(O)-$C_{1-6}$alkyl,

or
4 to 6 membered saturated heterocycle containing in the ring 1 or 2 heteroatoms independently selected from O, N and S;

$R^7$ is H,

$C_{1-6}$ alkyl, unsubstituted or substituted with one or more substituents independently selected from OH, halogen and O-$C_{1-6}$alkyl,
C(O)-$C_{1-6}$alkyl,
$COOC_{1-6}$alkyl, or
4 to 6 membered saturated heterocycle containing in the ring 1 or 2 heteroatoms independently selected from O, N and S;

$R^{8a}$ and $R^{8b}$ are independently from each other

H,
$C_{1-6}$alkyl and
oxo;

$R^9$ is H,

$C_{1-6}$ alkyl, unsubstituted or substituted with one or more substituents independently selected from OH, halogen and O-$C_{1-6}$alkyl,
C(O)-$C_{1-6}$alkyl,
$COOC_{1-6}$ alkyl, or
4 to 6 membered saturated heterocycle containing in the ring 1 or 2 heteroatoms independently selected from O, N and S;

$R^{10}$ is H,

$C_{1-6}$ alkyl, unsubstituted or substituted with one or more substituents independently selected from OH, halogen and O-$C_{1-6}$alkyl,
C(O)-$C_{1-6}$alkyl,

$COOC_{1-6}$ alkyl, or
4 to 6 membered saturated heterocycle containing in the ring 1 or 2 heteroatoms independently selected from O, N and S;

$R^{11}$ is H,

$C_{1-6}$ alkyl, unsubstituted or substituted with one or more substituents independently selected from OH, halogen and O-$C_{1-6}$alkyl,
C(O)-$C_{1-6}$alkyl,
$COOC_{1-6}$ alkyl, or 4 to 6 membered saturated heterocycle containing in the ring 1 or 2 heteroatoms independently selected from O, N and S;

$R^{12}$ is H or

$C_{1-6}$alkyl which is unsubstituted or substituted with one or more substitutents independently selected from OH and F;

$R^{13}$ is H or

$C_{1-6}$alkyl which is unsubstituted or substituted with one or more substitutents independently selected from OH and F;

$R^{14}$ is O-$C_{1-6}$ alkyl,

$C_{1-6}$alkyl or
$C_{3-6}$cycloalkyl;

$R^{15}$ is O-$C_{1-6}$alkyl,

$C_{1-6}$alkyl or
$C_{3-6}$cycloalkyl;

$R^{16}$ is OH,

O-$C_{1-6}$alkyl,
$C_{1-6}$alkyl or
$C_{3-6}$cycloalkyl;

$R^{17}$ is $C_{1-6}$alkyl or

$C_{3-6}$cycloalkyl;

$R^{18}$ is $NH_2$,

NH($C_{1-6}$alkyl), or
N($C_{1-6}$alkyl)$_2$;

$R^3$ is $-CH_2CR^{19a}R^{19b}CH_2$-T; $R^{19a}$ and $R^{19b}$ are independently from each other selected from

H,
OH, and halogen
or both together are =O;

T is

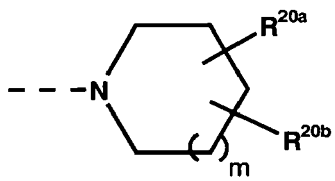

$R^{20a}$ and $R^{20b}$ are independently from each other selected from

H,
$C_{1-6}$alkyl and
$C_{3-6}$cycloalkyl;

m is 0 or 1;
n is 0, 1 or 2;
o is 1 or 2;
p is 1 or 2;
q is 0, 1 or 2 and
r is 0, 1 or 2.

**2.** The compound of claim 1 wherein A is a bond or methylene, unsubstituted or substituted with one $R^4$

**3.** The compound of claim 1 or 2 wherein $R^4$ is halogen or two alkyl chains attached to the same carbon atom of the alkylene, alkenylene or alkinylene form a 3-6 membered ring.

**4.** The compound of any one of claims 1 to 3 wherein $R^1$ and $R^2$ are independently from each other $C_{3-6}$alkyl.

**5.** The compound of any one of claims 1 to 4 wherein $R^3$ is $-CH_2CR^{19a}R^{19b}CH_2$-T wherein $R^{19a}$ is hydrogen and $R^{19b}$ is OH or halogen.

**6.** The compound of any one of claims 1 to 5 as a medicament.

**7.** The compound of any one of claims 1 to 5 as a melanocortin-4 receptor antagonist.

**8.** A compound of any one of claims 1 to 5 for use in the prophylaxis or treatment of a disorder, disease or condition responsive to the inactivation of the melanocortin-4 receptor in a mammal.

**9.** The compound of claim 8, wherein the disorder, disease of condition is selected from cachexia, muscle wasting, anorexia, anxiety, depression, amyotrophic lateral sclerosis (ALS), pain, nausea and emesis.

**10.** The compound of claim 9, wherein cachexia is selected from cancer cachexia, cachexia induced by chronic kidney disease (CKD) or cachexia induced by chronic heart failure (CHF) or cachexia induced by AIDS.

**11.** The compound of claim 9, wherein the anorexia is selected from anorexia nervosa or anorexia induced by radiotherapy or chemotherapy.

**12.** The compound of claim 9, wherein the pain is neuropathic pain.

**13.** Use of the compound of any one of claims 1 to 5 for the preparation of a medicament for the prophylaxis or treatment of disorders, diseases or conditions responsive to the inactivation of the melanocortin-4 receptor in a mammal.

**14.** The use of claim 13, wherein the disorder, disease of condition is selected from cachexia, muscle wasting, anorexia, anxiety, depression, amyotrophic lateral sclerosis (ALS), pain, nausea and emesis.

**15.** A pharmaceutical composition comprising a compound of any one of claims 1 to 5 and a pharmaceutically acceptable carrier.

Europäisches Patentamt
European Patent Office
Office européen des brevets

# EUROPEAN SEARCH REPORT

Application Number
EP 10 01 3420

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FR 2 862 971 A1 (SOD CONSEILS RECH APPLIC [FR]) 3 June 2005 (2005-06-03)<br>* page 1, paragraph 2 *<br>* page 1; compound (I) *<br>* page 33, line 19 - page 34, line 4 *<br>* examples 10, 19, 50, 61, 65 *<br>& WO 2005/056533 A1 (SOD CONSEILS RECH APPLIC [FR]; POITOUT LYDIE [FR]; BRAULT VALERIE [FR]) 23 June 2005 (2005-06-23)<br>----- | 1-15 | INV.<br>C07D235/16<br>C07D401/04<br>C07D401/12<br>C07D401/14<br>C07D403/04<br>C07D413/04<br>A61K31/4184<br>A61P1/08<br>A61P9/10<br>A61P25/24 |

TECHNICAL FIELDS SEARCHED (IPC)

C07D

The present search report has been drawn up for all claims

| Place of search | Date of completion of the search | Examiner |
|---|---|---|
| Berlin | 14 February 2011 | Hoepfner, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 10 01 3420

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2011

| Patent document cited in search report | Publication date | Patent family member(s) | Publication date |
|---|---|---|---|
| FR 2862971 A1 | 03-06-2005 | AT 482202 T | 15-10-2010 |
| | | BR PI0417001 A | 16-01-2007 |
| | | CA 2547391 A1 | 23-06-2005 |
| | | CN 1906174 A | 31-01-2007 |
| | | EP 1692114 A1 | 23-08-2006 |
| | | ES 2351148 T3 | 01-02-2011 |
| | | WO 2005056533 A1 | 23-06-2005 |
| | | JP 2007512294 T | 17-05-2007 |
| | | MX PA06005895 A | 27-06-2006 |
| | | RU 2369601 C2 | 10-10-2009 |
| | | US 2006281784 A1 | 14-12-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0074679 A
- WO 2005056533 A


### Non-patent literature cited in the description


- **A. KASK et al.** Selective antagonist for the melanocortin-4 receptor (HS014) increases food intake in free-feeding rats. *Biochem. Biophys. Res. Commun.,* 1998, vol. 245, 90-93
- **CHEN et al.** *Cell,* 1997, vol. 91, 789-798
- **S.Q. GIRAUDO et al.** Feeding effects of hypothalamic injection of melanocortin-4 receptor ligands. *Brain Research,* 1998, vol. 80, 302-306
- **D. HUSZAR et al.** *Cell,* 1997, vol. 88, 131-141
- **I. CORCOS et al.** HP-228 is a potent agonist of melanocortin receptor-4 and significantly attenuates obesity and diabetes in Zucker fatty rats. *Society for Neuroscience Abstracts,* 1997, vol. 23, 673
- **H. WESSELLS et al.** Synthetic Melanotropic Peptide Initiates Erections in Men With Psychogenic Erectile Dysfunction: Double-Blind, Placebo Controlled Crossover Study. *J. Urol.,* 1998, vol. 160, 389-393
- **OWEN MJ ; NEMEROFF CB.** Physiology and pharmacology of corticotrophin releasing factor. *Pharmacol Rev,* 1991, vol. 43, 425-473
- **SHIGEYUKI CHAKI et al.** *J. Pharm. Exp. Ther.,* vol. 304 (2), 818-26
- **MARKS D.L. et al.** Role of the central melanocortin system in cachexia. *Cancer Res.,* 2001, vol. 61, 1432-1438
- **CHEUNG W. et al.** Role of leptin and melanocortin signaling in uremia associated cachexia. *J. Clin. Invest.,* 2005, vol. 115, 1659-1665
- **CHEUNG W. et al.** Peripheral administration of the melanocortin-4 receptor antagonist NBI-12i ameliorates uremia-associated cachexia in mice. *J. Am. Soc. Nephrol.,* 2007, vol. 18, 2517-2524
- **BATRA A.K. et al.** Central melanocortin blockage attenuates cardiac cachexia in a rat model of chronic heart failure. *American Federation for Medical Research,* 2008
- **VAN CUTSEM E. ; ARENDS J.** The causes and consequences of cancer-associated malnutrition. *Eur. J. Oncol. Nurs.,* 2005, vol. 9 (2), 51-63
- **JOPPA M.A. et al.** Central infusion of the melanocortin receptor antagonist agouti-related peptide (AgRP(83-132)) prevents cachexia-related symptoms induced by radiation and colon-26 tumors in mice. *Peptides,* 2007, vol. 28, 636-642
- **LUDOLPH AC.** *Neuromuscul. Disord.,* 2006, vol. 16 (8), 530-8
- **ANNASAHEB S.K. et al.** Central administration of selective melanocortin 4 receptor antagonist HS014 prevents morphine tolerance and withdrawal hyperalgesia. *Brain Research,* 2007, vol. 1181, 10-20
- **CHEN, A.S. et al.** *Transgenic Res,* April 2000, vol. 9 (2), 145-54
- **MARKS, D.L. ; LING, N ; CONE, R.D.** *Cancer Res,* 15 February 2001, vol. 61 (4), 1432-8
- **PORSOLT R.D. ; BERTIN A. ; JAFFRE M.** Behavioral despair in rats and mice: strain differences and the effect of imipramine. *Eur. J. Pharmacol.,* 1978, vol. 51, 291-294
- **BORSINI F. ; MELI A.** Is the forced swimming test a suitable model for revealing antidepressant activity. *Psychopharmacol.,* 1988, vol. 94, 147-160
- **ZHAO, Y.H. et al.** Evaluation of Human Intestinal Absorption Data and Subsequent Derivation of a Quantitative Structure-Activity Relationship (QSAR) with the Abraham Descriptors. *Journal of Pharmaceutical Sciences,* 2001, vol. 90 (6), 749-784
- **DEFERME, S. ; MOLS, R. ; VAN DRIESSCHE, W. ; AUGUSTIJNS, P.** Apricot Extract Inhibits the P-gp-Mediated Efflux of Talinolol. *Journal of Pharmaceutical Sciences,* 2002, vol. 91 (12), 2539-48